# EUROPEAN PATENT SPECIFICATION

(11) **EP 3 108 846 B1**
(45) Date of publication and mention of the grant of the patent: **23.06.2021**
(21) Application number: 15752030.5
(22) Date of filing: 16.02.2015
(51) Int. Cl.: A61C 1/08, A61C 1/12, A61C 1/05, A61C 3/02

(54) **MEDICAL INSTRUMENT ELEMENT**
ELEMENT FÜR MEDIZINISCHES INSTRUMENT
ÉLÉMENT D'INSTRUMENT MÉDICAL

(30) Priority: 19.02.2014 JP 2014029319; 19.02.2014 JP 2014029320
(43) Date of publication of application: 28.12.2016
(73) Proprietor: J. Morita Manufacturing Corporation, Kyoto-shi, Kyoto 612-8213 (JP)
(72) Inventor: TANAKA Tsuyoshi, Kyoto-shi Kyoto 612-8213 (JP); NISHIMURA Mikinori, Kyoto-shi Kyoto 612-8213 (JP)
(74) Representative: Müller Hoffmann & Partner
(86) International application number: PCT/JP2015/054148
(87) International publication number: WO 2015/125736

(56) References cited:
- GB-A- 1 072 213
- JP-A- H1 024 052
- JP-A- H1 024 052
- JP-A- 2004 050 225
- JP-A- 2011 052 289
- JP-A- 2012 508 082
- US-A- 3 324 552
- US-A- 3 451 134
- US-A1- 2008 193 586
- US-A1- 2011 008 765
- US-B2- 8 323 677

## Description

### TECHNICAL FIELD

The present invention relates to a medical instrument element such as, for example, a dental handpiece device usable for dental care, a medical instrument, a medical instrument component, a medical handpiece, a method for producing the medical instrument element, and a medical cutting device, such as a dental handpiece device, that is usable to perform a medical care by allowing a cutting tool attached to a tip thereof to act on a tooth.

### BACKGROUND ART

Conventionally, a handpiece or the like including a cutting tool attached to a head at a tip thereof is widely used. Such a handpiece is operated as follows. The cutting tool is rotated by a built-in rotation driving device such as a micromotor or the like to cut a surgical operation target site. Recently in the field of dental treatment, a device that polymerizes photopolymerizable materials by photopolymerization radiation to perform a treatment on a tooth is in wide use. In such a device, a high-output LED is used as a light source. A dental handpiece including an LED light source is used so that a damaged site is irradiated and the dental care is made easily. A treatment device using laser light has been started to be used. Such a device also uses a laser light source.

For example, a contra-angle handpiece is usable to cut a treatment target site in a tooth in an oral cavity. In such a contra-angle handpiece or the like, the rotation rate of the cutting tool has been increased to, for example, five times greater because of the rotation rate of the micromotor. This causes a problem that the head including a tool holding unit that holds the cutting tool is warmed by the rotation.

In the case where the above-described LED or laser light source is incorporated into the handpiece, the light source generates heat. Recently, it is a problem to find how to sufficiently cool the handpiece.

In such a situation, JP-A-2004-520891 discloses forming a dental handpiece by metal injection molding (MIM).

However, metal injection molding does not provide a sufficiently high processing precision, especially for a head or the like that is required to have a complicated shape. Specifically, the processing precision provided by metal injection molding is about several hundred microns, with which the handpiece does not provide a desired level of performance.

Further prior art documents are US2011/0008765A1 disclosing a microfabricated tissue and US8,323,677B2 disclosing a therapeutic foam and JPH1024052 A disclosing a dental micro-motor handpiece.

### SUMMARY OF INVENTION

### TECHNICAL PROBLEM

The present invention made in light of the above-described problems has an object of providing a medical handpiece processed in a complicated manner with high precision so as to provide a desired level of performance.

### SOLUTION TO PROBLEM

The above objects are solved by the claimed matter according to the independent claim.

The present invention is directed to a medical handpiece, comprising a medium passage permitting a working medium to flow therein and including a bent portion at which a flow direction of the working medium is changed, the medical handpiece being formed by stack molding including a medium passage permitting a working medium to flow therein, by use of stack molding.

The working medium is a concept encompassing a fluid of any state such as liquid, gas, gel or the like, and also a medium that acts directly or indirectly on, namely, is usable to cool, cut, etc., a surgical operation target site or the like, for example, light.

The medium passage may be a passage formed by a hollow portion in a thick wall, a passage formed by a hollow path, or a passage accommodating another member such as a fiber or the like.

The stack molding refers to a formation method by which a layer is stacked on a previously formed layer, for example, stereolithography composite processing, stereolithography, powder sintering stacking, powder bonding stacking, thermal melting stacking, sheet stacking, inkjet stacking or the like.

The medical instrument element is a concept encompassing a medical instrument itself, a component included in the medical instrument, or a part of the medical instrument. The term "medical" encompasses "dental".

This invention provides a medical handpiece processed in a complicated manner with high precision so as to provide a desired level of performance.

This will be described in more detail. For example, a head or the like of a contra-angle handpiece has a compact shape from the viewpoint of surgical operability or the like at a surgical operation target site. Conventionally, in order to form a cooling path enclosing a tool holding portion holding a cutting tool in such a compact shape, linear holes are drilled from outside and such holes are connected to each other, or a component is divided along a plane passing a cooling path that is to be formed in the component and the cooling path is formed by cutting in an end surface of each of the divided parts. With such a method, the cooling path in which cooling water flows has a simple planar shape so as to be formed at an end surface of the separate component or a non-smooth shape formed by connecting a plurality of linear drilled holes. Therefore, the flow resistance is increased, and thus a cooling path having a high cooling effect is not provided.

According to the present disclosure, the medical instrument element including a medium passage that includes a bending portion at which the flow direction of the working medium is changed is formed by stack molding, by which a layer is stacked on a previously formed layer. In this case, for example, even a medium passage having a complicated three-dimensional shape is formed accurately. Unlike in the case where holes are drilled from outside to form a medium passage inside, an unnecessary opening is not formed. Thus, a medical instrument element having a high processing precision and a high sealability is provided.

For example, the medium passage formed by stack molding may have a desired shape . Therefore, as compared with the case where the medium passage is formed by a combination of a plurality of linear through-holes, the path has a lower path resistance and a higher flow efficiency. This increases the work efficiency provided by the working medium. Specifically, with the stereolithography composite processing according to the present invention, the processing precision is in the order within ± several micrometers. Therefore, high precision processing is performed, and thus a predetermined level of performance is exhibited.

In an example, the working medium may be formed of a cooling medium; and the medium passage may be a cooling path that is located in the vicinity of an operation portion warmed by operating and cools the operation portion by the cooling medium flowing in the medium passage.

The cooling medium may be cooling liquid such as cooling water or gas such as cooling air. Alternatively, a cooling medium which has flown in the cooling path may be supplied to the surgical operation target site.

This disclosure provides a medical instrument element that includes a cooling path having a higher cooling efficiency and has a high processing precision.

Specifically, even a cooling path having a complicated three-dimensional shape is formed to have a smooth shape having a low path resistance. This increases the cooling efficiency provided by the cooling medium.

In the case where, for example, the medium passage is formed by incorporating a component, different from the components included in the medical instrument element, into the medical instrument element, a tiny hollow member as the different component needs to be formed with high precision and incorporated with high precision such that working medium does not leak. In the case where the medium passage is formed by stack molding, even if such a different member is used to form the medium passage, the stack molding may be performed in the state where the different member is incorporated. Therefore, the medical instrument element is formed with high precision with no step of incorporating the medium passage formed of the different member into the medical instrument element. Needless to say, in the case where the medium passage is formed in the medical instrument element itself by molding, the number of components is decreased and the structure is suppressed from being complicated as compared with the case where such a different component is incorporated.

In an example, the medical instrument element may be formed by stereolithography composite processing used as the stack molding, the stereolithography composite processing being performed by a repetition of stereolithography and cutting processing.

This disclosure provides a medical instrument element formed to have a desired shape with high precision.

This will be described in more detail. A powder material such as a metal material or the like is squeezed on a top surface of a stacked body formed so far, and the squeezed powder is sintered with laser light to form a layer having a thickness of, for example, 5 micrometers. This is stereolithography. The stereolithography and cutting processing, by which the layer formed by stereolithography is subjected to high-speed cutting, are performed. Therefore, on a stacked body formed so far, another layer is stacked by stereolithography and is shaped with high precision by high-speed cutting. Thus, a desired shape is realized with higher precision. The powder material may be a resin material, a ceramic material or the like as well as a metal material described above.

For example, a cycle of performing stereolithography a predetermined number of times and then performing cutting processing may be repeated. For example, stereolithography may be performed for five layers before the cutting processing. Alternatively, a cycle of performing stereolithography and cutting processing alternately may be repeated.

In an example, a difficult-to-cut material may be used as a material for the element.

The difficult-to-cut material may be a metal material considered to be difficult to be cut in usual cutting processing, for example, titanium, stainless steel or the like.

Owing to this disclosure, even a difficult-to-cut material considered to be difficult to be cut due to a factor of a material strength or the like may be used to form a desired shape with high precision. A reason for this is that stereolithography of squeezing and sintering a powder material to stack metal layers having a desired shape, and cutting processing of cutting the formed layers with higher precision, are repeated.

In an embodiment of the present invention, the medium passage may be formed to have a cross-sectional diameter of 0.6 mm or less.

For example, in consideration of leak or the like of a flowing working medium, it is desired to form the medium passage by forming a hole. With such a method, it is impossible to form a medium passage having a cross-sectional diameter of 0.6 mm or less with high precision. However, in the case where stereolithography of stacking layers having a desired shape by sintering and cutting processing of cutting the formed layers with higher precision are repeated, even a medium passage having a cross-sectional diameter of 0.6 mm or less is formed with a desired shape with high precision.

In an example, the medium passage may be porous with microscopic pores.

This example prevents the strength of the medical instrument element from being decreased due to the provision of the medium passage.

The present disclosure is also directed to a medical instrument including the above-described medical instrument element.

The medical instrument may be a medical handpiece, a medical laser handpiece or the like partially or entirely formed by stack molding.

Owing to this disclosure, a medical instrument that is not much limited by ease of incorporation or the like and is suitable to a desired performance is formed with high precision.

The present disclosure is also directed to a medical instrument component including the above-described medical instrument element, the medical instrument element forming a part of a medical instrument.

The medical instrument component may be, for example, a component included in a medical instrument such as a medical handpiece, a medical laser handpiece or the like. Specifically, the medical instrument component may be a connection portion connecting the medical handpiece and a tube, a head of the medical handpiece, an air turbine handpiece body of the medical handpiece, a bladed wheel, or the like. The medical instrument component may be a medical micromotor handpiece, a medical scaler handpiece, a three-way syringe, or a medical photopolymerization radiation

Owing to this disclosure, for example, a medical instrument component that is not much limited by processability or the like and is suitable to a desired performance is formed with high precision.

The present invention is directed to a medical handpiece including the above-described medical instrument element; and the operation portion acting as a motive power transmission element transmitting a motive power or a guide element guiding an LED, a laser light source itself, or another member another member.

The motive power transmission element may be a transmission mechanism such as a gear, a bladed wheel or the like, and the guide element may be a guide mechanism such as a bearing or the like.

Owing to this invention, a highly precise medium passage is formed even in a medical handpiece including an operation portion having a complicated structure. Therefore, a medical handpiece having a high work efficiency provided by a working medium such as a cooling effect provided by cooling water is provided.

The present disclosure is also directed to a medical cutting device including a tool holding portion holding a cutting tool cutting a surgical operation target site; a head including the tool holding portion; a rotation driving transmission portion causing the cutting tool to rotate; a housing cooling path permitting a cooling medium to flow to the rotation driving transmission portion; and a housing accommodating the rotation driving transmission portion. The head includes at least a head cooling path that is formed to enclose the tool holding portion in the head, is in communication with the housing cooling path, and permits the supplied cooling medium to flow therein.

The cutting tool is a tool cutting a tooth by rotating. A rotation driving transmission portion rotating the cutting tool may be a gear mechanism portion coupled with a device electrically rotating the cutting tool, for example, a micromotor, or a bladed wheel rotating the cutting tool by air.

The medical cutting device including the head and the housing encompasses a medical cutting device having a so-called contra-angle handpiece structure in which a small-diameter neck is provided between the head and the housing and a tool axis and a grip axis form a predetermined angle; a medical cutting device of a straight handpiece type which includes a head including a head main body and a neck integral with each other and in which the tool axis and the grip axis are coaxial with each other; a medical cutting device that includes a head and a housing including a housing main body and a neck integral with each other; a medical cutting device including only a head and a housing, or a medical cutting device in which a part of the housing is a head.

The medical cutting device may be a medical handpiece or a medical laser handpiece. The medical cutting device may be a micromotor handpiece using a micromotor as a driving source, or an air turbine handpiece using compressed air as a driving source.

The head cooling path may be directly connected with, so as to be communicated with, the housing cooling path. Alternatively, for example, a neck cooling path may be provided in the neck so that the head cooling path and the housing cooling path are communicated with each other via the neck cooling path.

The head cooling path may be a path formed in the thick wall of the head, or a path formed of a different member. The head cooling path may be a part of a circulation path circulating the cooling medium flowing therein, or a path of a non-circulation path not circulating the cooling medium flowing therein.

The cooling medium may be liquid such as cooling water or the like, powder or gel.

Owing to this disclosure, the head warmed by the rotation of the cutting tool held by the tool holding portion or the operation of the driving force transmission mechanism is cooled.

This will be described in more detail. In, for example, a contra-angle handpiece cutting a treatment target site in a tooth in an oral cavity, the rotation rate of the cutting tool has been increased to, for example, five times greater because of the rotation rate of the micromotor. This causes a problem that the head including a tool holding unit that holds the cutting tool is warmed by the rotation of the cutting tool or the operation of the driving force transmission mechanism.

In such a situation, a dental contra-angle handpiece including a water supply groove or an air supply groove for cooling that has a partially annular shape in the vicinity of the head has been proposed. According to this idea, water is supplied to the cooling water supply groove and air is supplied to the air supply groove to cool the area in the vicinity of the warmed head (Japanese Utility Model Registration No. 3125885).

However, this does not cool the head warmed by the rotation of the cutting tool held by the tool holding portion or the operation of the driving force transmission mechanism. A reason for this is as follows. Provision of a cooling mechanism in the head requires the head to be processed to provide a cooling portion that is precise and effective for cooling without enlarging the head. This is not realized.

By contrast, according to the present disclosure, the cooling medium which has flown in the housing cooling path is caused to flow in the head cooling path formed to enclose the tool holding portion in at least the head. Thus, the cooling medium flowing in the head cooling path and the warmed head perform heat exchange. Thus, the head warmed by the rotation of the cutting tool is cooled efficiently.

In an embodiment of the present disclosure, one of two ends of the head cooling path may be connected with the housing cooling path; the other of the two ends of the head cooling path may be a water supply opening that is opened in a bottom portion of the head cooling path to allow water to be supplied to the surgical operation target site; and the cooling medium may be cooling water supplied from the water supply opening to the surgical operation target site to cool the surgical operation target site.

Owing to this disclosure, the surgical operation target site is cut while the cooling water is supplied from the water supply opening to the surgical operation target site and the head is cooled by the cooling water. According to the present invention, the cooling water which has flown in the head cooling path is supplied to the surgical operation target side. In this case, as compared with the case where the head cooling path provided in the head and the water supply path are separately provided, the medical cutting device has a simpler structure. Therefore, the number of components is decreased, and the medical cutting device is compact.

In an example, a plurality of the head cooling paths may be located parallel to each other in an axial direction of the cutting tool.

This example increases the cooling efficiency provided by the cooling medium.

This will be described in more detail. The plurality of head cooling paths are located parallel to each other in an axial direction of the cutting tool. Owing to this, the surface area size of the head cooling path is increased. This increases the thermal exchange efficiency with the cooling water flowing in the head cooling path, and thus increases the efficiency of cooling the head.

In an example, the head may include a cooling air path permitting cooling air to flow therein; and the cooling air which has flown in the cooling air path and the cooling water may be mixed together and supplied.

Owing to this invention, the cooling water and the cooling air are mixed and sprayed from the water supply opening. Thus, the surgical operation target site is cooled efficiency.

In an example, the cooling air path may be porous with microscopic pores.

This example prevents the strength of the head from being decreased due to the provision of the cooling air path.

In an example, the medical cutting device may be formed by stack molding.

The stack molding refers to a formation method such as, for example, stereolithography composite processing, stereolithography, powder sintering stacking, powder bonding stacking, thermal melting stacking, sheet stacking, inkjet stacking or the like.

Owing to this example, the medical cutting device having a complicated shape is formed without forming any unnecessary opening.

This will be described in more detail. In the case where the head cooling path enclosing the tool holding portion is formed by general drilling or the like, drilling needs to be performed linearly. Therefore, a plurality of linear through-holes are formed in combination. In this case, an unnecessary opening is formed. In the case where stereolithography is used, for example, even the head including the head cooling path enclosing the tool holding portion is formed by molding with no provision of such an unnecessary opening.

In the case where, for example, the head cooling path is formed by incorporating a component different from the components included in the head, which is desired to be compact, into the head, a tiny hollow member as the different component needs to be formed with high precision and incorporated with high precision such that cooling medium does not leak. In the case where stack molding is used, even if such a different member is used to form the head cooling path, the stack molding may be performed in the state where the different member is incorporated. Therefore, the head is formed with high precision with no step of incorporating the head cooling path formed of the different member into the head. Needless to say, in the case where the head cooling path is formed in a component forming the head by molding, the number of components is decreased and the structure is suppressed from being complicated as compared with the case where such a different component is incorporated.

For example, the head cooling path formed by stack molding may have a desired shape . Therefore, as compared with the case where the head cooling path enclosing the tool holding portion is formed by a combination of a plurality of linear through-holes, the cooling path has a lower path resistance and a higher flow efficiency. This increases the cooling efficiency provided by the cooling medium. Regarding an air turbine handpiece, a handpiece having a low driving air resistance and a good energy efficiency is formed.

### ADVANTAGEOUS EFFECTS OF INVENTION

The present invention provides a medical handpiece processed in a complicated manner with high precision so as to provide a desired level of performance.

### BRIEF DESCRIPTION OF DRAWINGS

[FIG. 1] FIG. 1 is a front view of a dental instrument.
[FIG. 2] FIG. 2 shows a contra-angle handpiece.
[FIG. 3] FIG. 3 shows the contra-angle handpiece.
[FIG. 4] FIG. 4 shows a head housing.
[FIG. 5] FIG. 5 shows a stack molding method for the head housing.
[FIG. 6] FIG. 6 is a flowchart of the stack molding method.
[FIG. 7] FIG. 7 shows a first layer group and a second layer group of the head housing formed by the stack molding method.
[FIG. 8] FIG. 8 shows the stack molding method for the first layer group.
[FIG. 9] FIG. 9 shows a third layer group and a fourth layer group of the head housing formed by the stack molding method.
[FIG. 10] FIG. 10 shows a fifth layer group and a sixth layer group of the head housing formed by the stack molding method.
[FIG. 11] FIG. 11 is a cross-sectional view of another head housing.
[FIG. 12] FIG. 12 shows a main part of a dental air turbine handpiece.
[FIG. 13] FIG. 13 is a cross-sectional view in a planar direction of the dental air turbine handpiece.
[FIG. 14] FIG. 14 is a perspective view of a rotor included in the dental air turbine handpiece.
[FIG. 15] FIG. 15 is a cross-sectional view of the entirety of a handpiece in an assembled state.
[FIG. 16] FIG. 16 is a cross-sectional view of a main connection portion of the handpiece in a separate state.
[FIG. 17] FIG. 17 is a cross-sectional view of a dental handpiece.
[FIG. 18] FIG. 18 is a cross-sectional view of a main part of the dental handpiece.
[FIG. 19] FIG. 19 is an enlarged vertical cross-sectional view of a three-way syringe as seen from the side.
[FIG. 20] FIG. 20 shows a dental air scaler.
[FIG. 21] FIG. 21 provides cross-sectional views of the dental air scaler.
[FIG. 22] FIG. 22 is a front view of a laser radiation chip.
[FIG. 23] FIG. 23 is a partially cut enlarged view of the laser radiation chip.
[FIG. 24] FIG. 24 is an enlarged cross-sectional view of a tip end of the laser radiation chip.

### DESCRIPTION OF EMBODIMENTS

An embodiment of the present invention will be described with reference to the drawings.

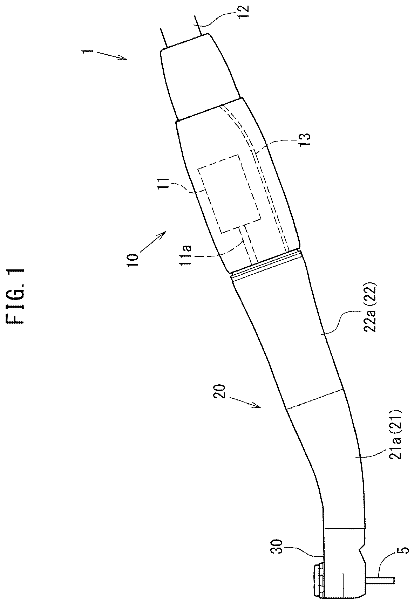

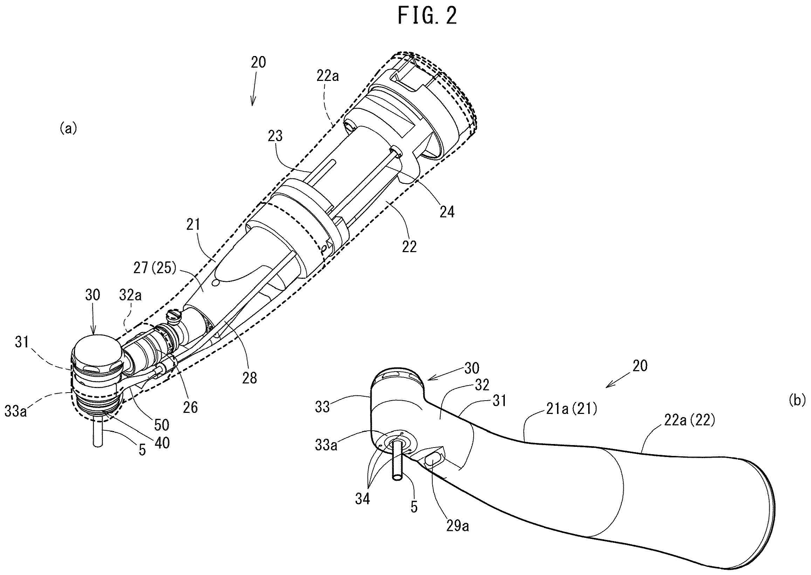

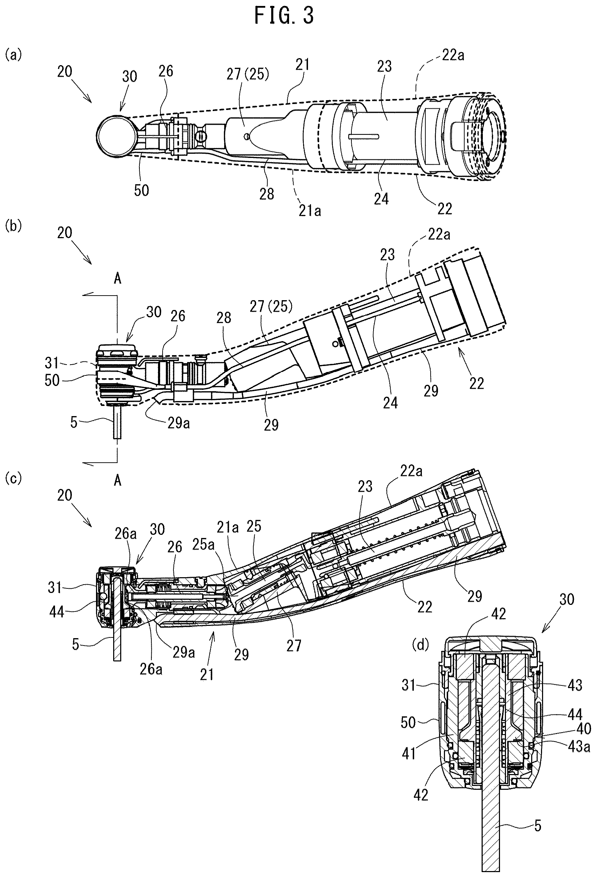

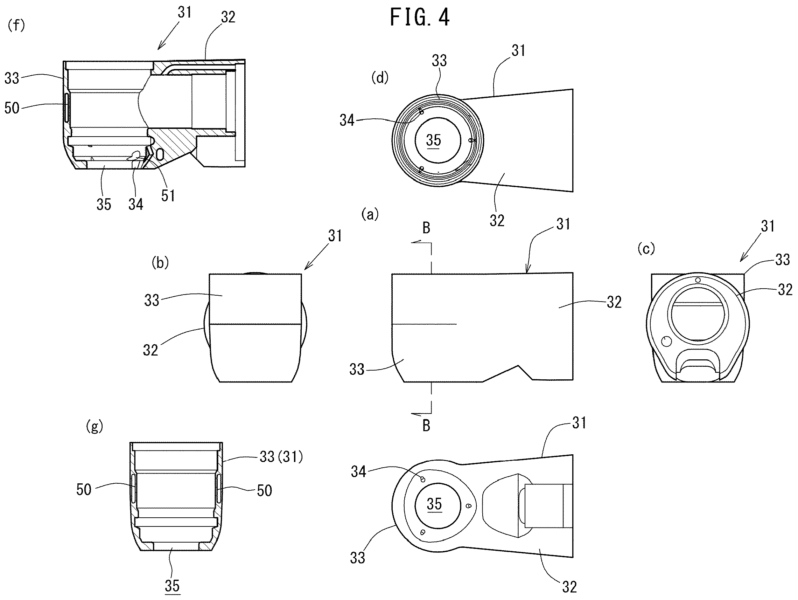

FIG. 1 is a front view of a micromotor handpiece 1 as a dental care instrument. FIG. 2 shows an inner structure of a contra-angle handpiece 20, which is a part of the micromotor handpiece 1. FIG. 3 shows the contra-angle handpiece 20. FIG. 4 shows a head housing 31 of the contra-angle handpiece 20.

The above will be described more specifically. FIG. 2(a) is a perspective view of the contra-angle handpiece 20 as seen from the top side. FIG. 2(b) is a perspective view of the contra-angle handpiece 20 as seen from the bottom side. FIG. 2 (a) shows an outer casing (head housing 31, neck cover 21a, grip cover 22a) of the contra-angle handpiece 20 as being transparent. A cooling path 50 is a space formed in a thick wall of the head housing 31 shown as being transparent. In FIG. 2(a), the cooling path 50 is shown as being positively present for easier understanding.

FIG. 3(a) is a plan view of the contra-angle handpiece 20. FIG. 3(b) is a front view of the contra-angle handpiece 20. FIG. 3(c) is a vertical cross-sectional view of the contra-angle handpiece 20. FIG. 3(d) is a cross-sectional view of the contra-angle handpiece 20 taken along line A-A shown in FIG. 3(b). Like FIG. 2(a), FIG. 3 (a) shows the outer casing of the contra-angle handpiece 20 as being transparent. Like in FIG. 2(a), in FIG. 3(b), the cooling path 50, which is a space formed in the thick wall of the head housing 31, is shown as being positively present for easier understanding.

FIG. 4(a) is a front view of the head housing 31, FIG. 4(b) is a left side view thereof, FIG. 4 (c) is a right side view thereof, FIG. 4(d) is a plan view thereof, FIG. 4(e) is a bottom view thereof, FIG. 4(f) is a vertical cross-sectional view thereof, and FIG. 4(g) is a cross-sectional view thereof taken along line B-B shown in FIG. 4(a).

As shown in FIG. 1, the micromotor handpiece 1 as a dental care instrument in this embodiment includes the contra-angle handpiece 20, and a motor unit 10 including a built-in micromotor 11. The contra-angle handpiece 20 and the motor unit 10 are detachably coupled with each other.

The motor unit 10 includes the micromotor 11 driving and rotating a cutting rotation tool 5 detachably attached to a head 30, a first cooling pipe 24 of the contra-angle handpiece 20 described below, and a second cooling pipe 13 connected with a water supply path of a hose 12 to permit cooling water to flow therein.

A rear end of the motor unit 10 is coupled with a power supply lead supplying power to the micromotor 10 built in the motor unit 10, a power supply lead supplying power to a light source irradiating a damaged site described below, and the hose 12 having a supply path for cooling air provided therein.

The contra-angle handpiece 20 includes the head 20, a neck 21 linked with the head 30 and having a small diameter, and a body 22 linked with the neck 21, which are located in this order from a tip end of the contra-angle handpiece 20. An operator grips the neck 21 and the body 22 with his/her fingers.

The neck 21 and the body 22 are covered with a cylindrical outer cover 22a formed of a metal material. The neck 21 and the body 22 accommodate a first driving transmission shaft 23 coupled with an output shaft 11a of the micromotor 11 included in the motor unit 11 connected with the contra-angle handpiece 20 and also accommodate the first cooling pipe 24 permitting cooling water to flow therein.

The first driving transmission shaft 23 is axially rotatable in the neck 21 and the body 22 along with a rotation of the output shaft 11a, of the micromotor 11, coupled with the first driving transmission shaft 23.

The neck 21 is covered with the neck outer cover 21a, which is cylindrical and formed of a metal material. The neck 21 accommodates a second driving transmission shaft 25 and a third driving transmission shaft 26, which are linked with each other so as to be bendable and axially rotatable by a bevel gear 25a. The second driving transmission shaft 25 and the third driving transmission shaft 26 are located in this order from a rear end of the neck 21.

The second driving transmission shaft 25 is enclosed by a gear box 27, and a front bevel gear 26a is secured to a tip end of the third driving transmission shaft 26. The neck 21 accommodates a second cooling pipe 28 to the side of the second driving transmission shaft 25 and the gear box 27. The second cooling pipe 28 is connected with the first cooling pipe 24 located in the grip 22.

The neck 21 and the grip 22 accommodate a light guide path 29 usable to project illumination light emitted by an LED (not shown) located inside the motor unit 10 toward a surgical operation target site through a projection opening 29a provided on the bottom side of the neck 21.

The head 30 includes the head housing 31 continued from the neck cover 21a to form an outer casing of the head 30, and a tool holding portion 40 located inside the head housing 31. The tool holding portion 40 holds the cutting rotation tool 5 such that the cutting rotation tool 5 is rotatable around an axis perpendicular to an axis of the third driving transmission shaft 26 and is detachable from the tool holding portion 40.

The head housing 31 includes a main body-side cylindrical portion 32 and a tip end-side cylindrical portion 33. The main body-side cylindrical portion 32 is provided on a rear end side of the head housing 31 and extends in a longitudinal direction to be continued from the neck cover 21a of the neck 21. The tip end-side cylindrical portion 33 extends in a direction substantially perpendicular to the main body-side cylindrical portion 32, namely, in an up-down direction in FIG. 4 (f) and FIG. 4(g), and has a bottom surface having a bottom surface opening 35. The main body-side cylindrical portion 32 and the tip end-side cylindrical portion 33 form an inner space that is substantially T-shaped in a vertical cross-section.

The tool holding portion 40 includes a substantially cylindrical capsule 41 provided inside the tip end-side cylindrical portion 33, a rotor shaft 43 rotatable via top and bottom bearings 42 located inside the capsule 41, and a chuck 44 attached to the rotor shaft 43 to grip the cutting rotation tool 5.

At a bottom end of the rotor shaft 43, a rotor gear 43a is formed. The rotor gear 43a is in engagement with the front bevel gear 26a formed at the tip end of the third driving transmission shaft 26. Thus, along with a rotation of the third driving transmission shaft 26, the third driving transmission shaft 26, chuck 44 and the cutting rotation tool 5 are rotated.

Inside the tip end-side cylindrical portion 33, which includes a thick wall, of the head 30, the cooling path 50 is formed so as to enclose the tool holding portion 40. The tool holding portion 40 is located inside the tip end-side cylindrical portion 33.

A base end of the cooling path 50 is in communication with a tip end of the second cooling pipe 28 located in the neck 21. A middle portion of the cooling path 50 extends in a spiral manner coaxially with the tool shaft so as to enclose a tip end of the tool holding portion 40. A tip end of the cooling path 50 is a water supply opening 34 formed in a bottom surface 33a of the tip end-side cylindrical portion 33.

Inside the head 30, a cooling air path 51 (see FIG. 4(f)) usable to introduce cooling air is formed. A tip end of the cooling air path 51 is connected with a middle portion of the cooling path 50 to form a meeting portion 52. Cooling water flowing in the cooling path 50 is supplied from the water supply opening 34 together with cooling air introduced from the cooling air path 51.

The cooling path 50 may be formed to have a cross-sectional diameter of 0.6 mm or less, and the cooling air path 51 may be porous with microscopic pores.

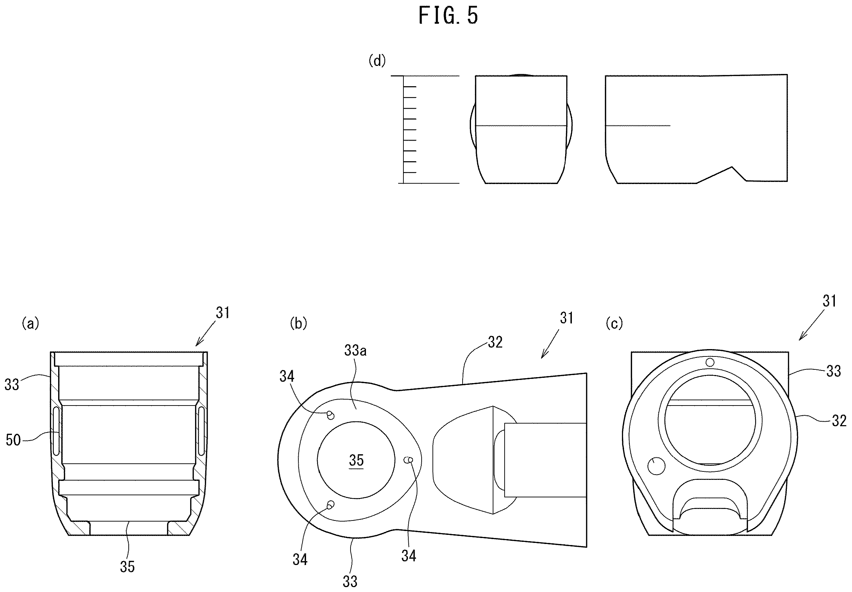

The head housing 31 including the cooling path 50 formed in the thick wall of the tip end-side cylindrical portion 33 as described above is formed by a stack molding method described below. Hereinafter, with reference to FIG. 5 through FIG. 13, the formation of the head housing 31 by stack molding will be described in detail.

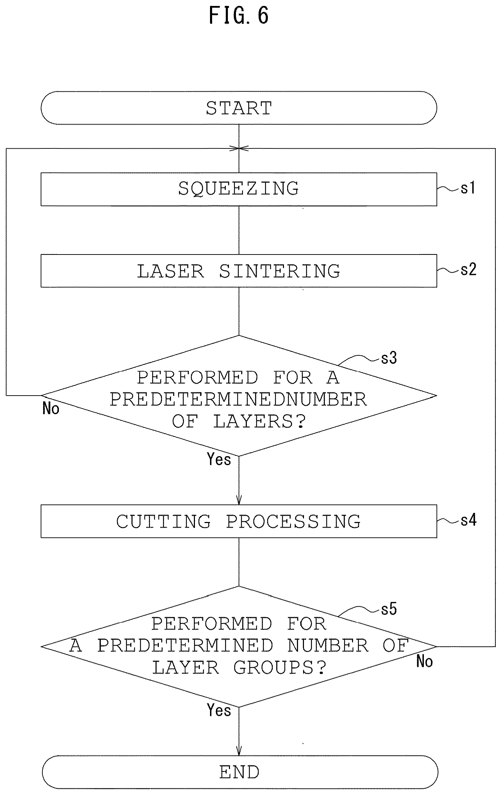

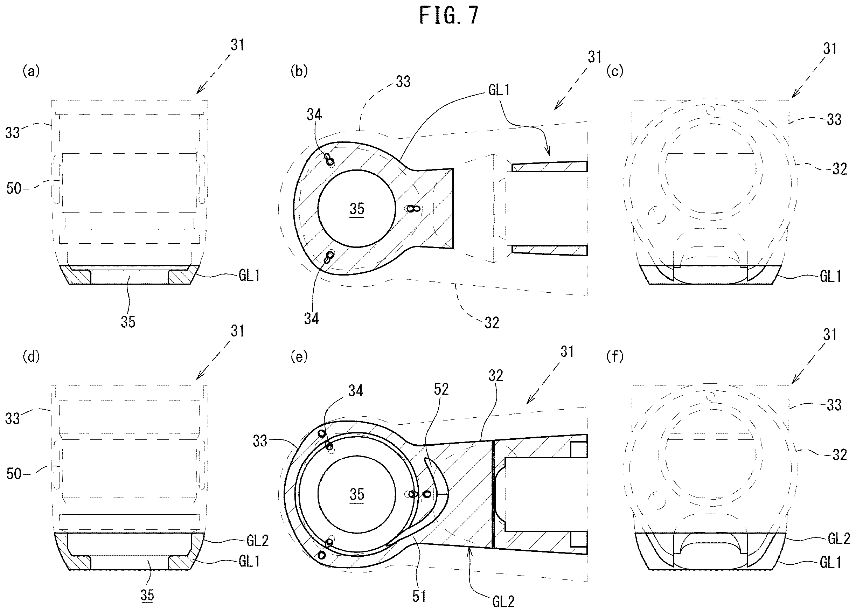

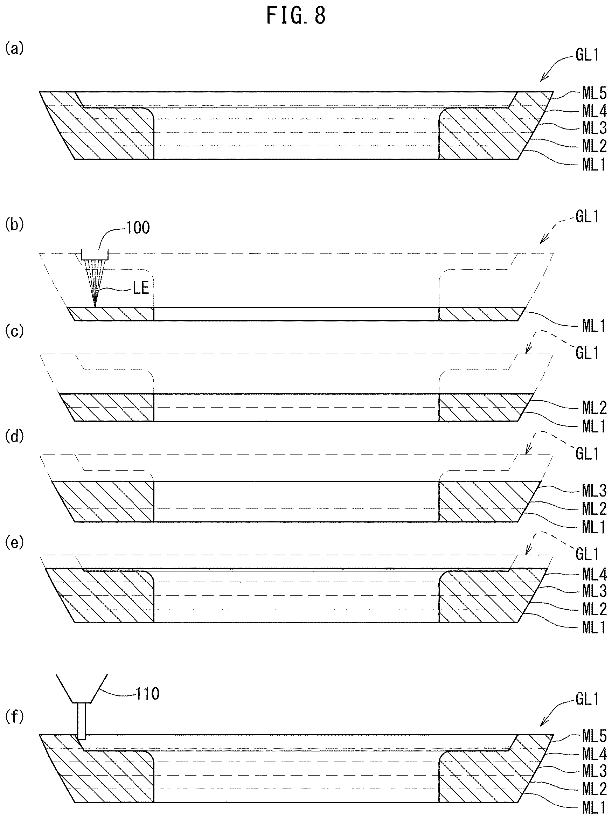

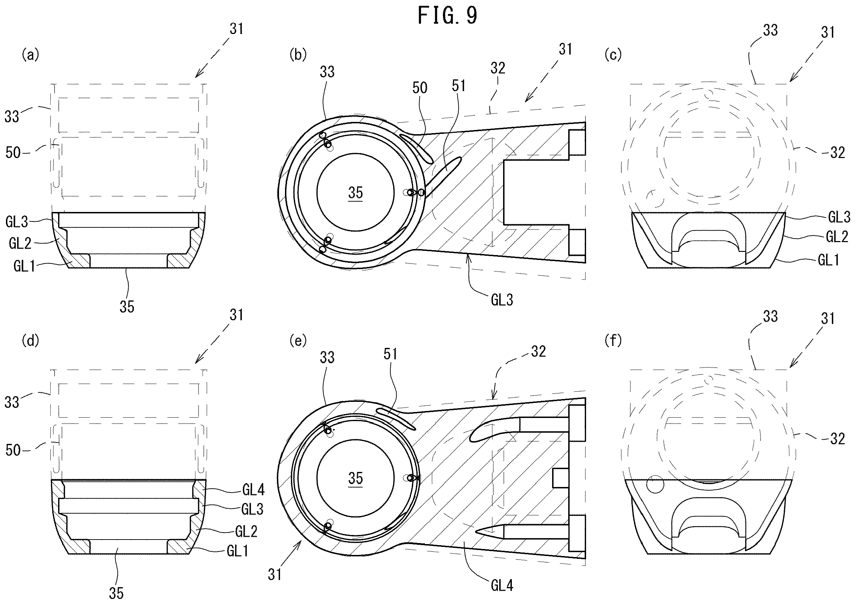

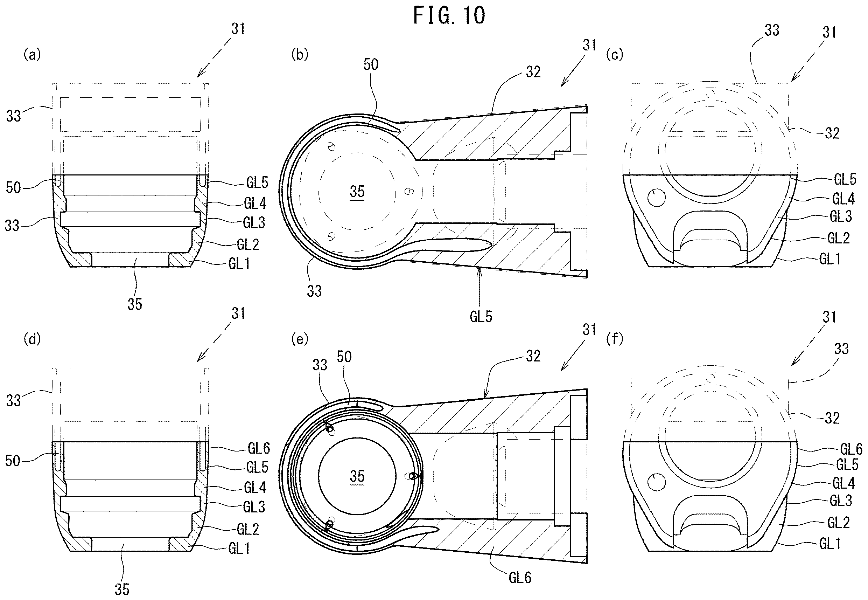

FIG. 5 shows the stack molding method for forming the head housing 31. FIG. 6 is a flowchart showing the stack molding method. FIG. 7 shows a first layer group GL1 and a second layer group GL2 of the head housing 31 formed by the stack molding method. FIG. 8 shows the stack molding method for forming the first layer group GL1. FIG. 9 shows a third layer group GL3 and a fourth layer group GL4 of the head housing 31 formed by the stack molding method, like FIG. 7. FIG. 10 shows a fifth layer group GL5 and a sixth layer group GL6 of the head housing 31 formed by the stack molding method.

The above will be described in more detail. FIG. 5(a) is a cross-sectional view, as seen from the side, of the tip end-side cylindrical portion 33 of the head housing 31. FIG. 5(b) is a bottom view of the head housing 31. FIG. 5 (c) is a view of the head housing 31 as seen from a base end thereof, namely, is a right side view thereof. FIG. 5(d) is a schematic view showing layer groups used to form the head housing 31 by the stack molding method.

FIG. 7(a) is a cross-sectional view, as seen from the side, of the tip end-side cylindrical portion 33 in a state where the first layer group is formed by molding. FIG. 7(b) is a plan view of the head housing 31 in the state of FIG. 7(a). FIG. 7(c) is a right side view of the head housing 31 in the state of FIG. 7(a). In FIG. 7(a), FIG. 7(b) and FIG. 7(c), the dashed line shows the state that is to be obtained when the molding is finished. FIG. 7(d), FIG. 7(e), FIG. 7(f), FIG. 9 and FIG. 10 each show the state where another layer group(s) is(are) formed, in correspondence with FIG. 7(a), FIG. 7(b) and FIG. 7(c).

FIG. 8 provides cross-sectional views showing how metal layers ML included in the first layer group are stacked.

In the following example, the head housing 31 is formed by molding by use of metal stereolithography composite processing.

The metal stereolithography composite processing performed to form the head housing 31 is as follows. Squeezing on a metal power and laser sintering, by which the squeezed metal powder is sintered by laser light, are repeated until a predetermined number of layers are formed, and then, high-speed cutting is performed. This procedure is repeated to form the head housing 31. In the following example, squeezing and laser sintering are repeated until five layers are formed, and then, high-speed cutting is performed. A layer formed by repeating squeezing and laser sintering will be referred to as a "metal layer ML". A layer group that is obtained by repeating squeezing and laser sintering to form a stack of five metal layers ML and then performing high-speed cutting by a cutting device 110 will be referred to as a "layer group GL".

In the following example, the layers included in the head housing 31 are stacked from the side of a bottom surface, and ten layer groups are stacked. The number of layers included in one layer group GL, and the number of the layer groups, are not limited to those described above. Any appropriate number of layers, and any appropriate number of layer groups, may be used.

The head housing 31 is formed by molding by use of metal stereolithography composite processing as follows. As shown in FIG. 6, a metal powder is squeezed to have a predetermined thickness (step s1). The squeezed metal powder squeezed having the predetermined thickness is scanned with laser light Le oscillated by a laser radiation device 100 and thus sintered (see FIG. 8(b)), so as to have a predetermined shape. Thus, a metal layer ML having a predetermined thickness is formed (step s2).

The squeezing (step s1) and laser sintering (step s2) are repeated until a predetermined number of metal layers (in this embodiment, five layers) are formed (step s3: No). The predetermined number of stacked metal layers ML are used to form a layer group GL (step s3: Yes). Then, a surface of the layer group GL having a predetermined shape is cut by the cutting device 110. Thus, the layer group GL having high precision is formed (step s4) .

The steps of repeating the squeezing (step s1) and laser sintering (step s2) until a predetermined number of layers are formed (step s3: Yes) and performing the cutting by the cutting device 110 (step s4) are considered as one cycle. This cycle is repeated until a predetermined number of layer groups (in this embodiment, ten layer groups) are formed (step s5). Thus, the head housing 31 having a desired shape is formed.

Specifically, a first metal layer ML1 of the first layer group GL1 forms the bottom surface of the head housing 31. As shown in FIG. 7(b), the water supply opening 34 formed at the tip end of the cooling path 50 and opened in the bottom surface 33a of the tip end-side cylindrical portion 33, the bottom surface 33a of the tip end-side cylindrical portion 33 having the bottom surface opening 35 permitting the tool holding portion 40 to be exposed, and an area including a bottom surface and the vicinity thereof of the main body-side cylindrical portion 32 are formed.

In a second layer group GL2, as shown in FIG. 7(d), FIG. 7 (e) and FIG. 7(f), the meeting portion 52 of the cooling path 50 connected with the water supply opening 34 formed in the first metal layer GL1 and the cooling air path 51 is formed around a rear end of the bottom surface opening 35 so as to be substantially U-shaped as seen in a plan view.

In a third layer group GL3, as shown in FIG. 9(a), FIG. 9(b) and FIG. 9(c), an area including the tip end and the vicinity thereof of the cooling path 50 directed toward the meeting portion 52 formed in the layer group GL2, and an area including the tip end and the vicinity thereof of the cooling air path 51 also directed to the meeting portion 52 formed in the layer group GL2, are formed as crossing each other.

In a fourth layer group GL4, as shown in FIG. 9(d), FIG. 9(e) and FIG. 9(f), a part of the cooling path 50, and a part of a bottom surface of the main body-side cylindrical portion 32 extending in the horizontal direction, are formed.

In a fifth layer group GL5, as shown in FIG. 10(a), FIG. 9(b) and FIG. 9(d), the cooling path 50 is formed so as to enclose the bottom surface opening 35, and a hollow portion of the main body-side cylindrical portion 32 and a hollow portion of the tip end-side cylindrical portion 33 are formed in communication with each other.

In a sixth layer group GL6, as shown in FIG. 10(d), FIG. 10(e) and FIG. 10(f), a top portion of the cooling path 50 enclosing the bottom surface opening 35 is formed, and a hollow portion of the main body-side cylindrical portion 32 and a hollow portion of the tip end-side cylindrical portion 33 are formed in communication with each other.

In this manner, in each layer group GL, the hollow portion and the solid portion are formed in accordance with the structure of the respective layer group. Such formation is performed for a predetermined number of layer groups. As a result, the head housing 31 including a stack of a predetermined number of layer groups GL each including a predetermined number of metal layers ML and having a hollow portion in the thick wall is formed.

The dental care instrument 1 includes the head housing 31 formed as described above. In the thick wall of the head housing 31 of the dental care instrument 1, the cooling path 50 is formed so as to enclose the tool holding portion 40. In the cooling path 50, cooling water which has flown in the second cooling pipe 28, the first cooling pipe 24 and the second cooling pipe 13 flows. The head 30, which is warmed by the rotation of the cutting rotation tool 5 held by the tool holding portion 40 and the operation of the third driving transmission shaft 26 and the front bevel gear 26a, and the cooling water flowing in the cooling path 50, perform heat exchange. Thus, the head 30 is cooled.

The base end of the cooling path 50 is connected with the second cooling pipe 28, and the tip end of the cooling path 50 is the water supply opening 34 opened in a bottom portion of the cooling path 50 such that water is supplied to the surgical operation target site. Owing to such an arrangement, the head 30 is cooled with the cooling water, and the cooling water is supplied to the surgical operation target site from the water supply opening 34 while the surgical operation target site is cut by the cutting rotation tool 5. As a result, the water is warmed by the head 30 to a temperature close to the body temperature and is supplied to the surgical operation target site. Thus, the water is supplied at a temperature that is neither too cold and stimulative nor too hot for the damaged site.

The cooling path 50 provided in the head 30 is formed in the thick wall of the head housing 31. Owing to such an arrangement, as compared with the case where a path for supplying water and a path for cooling water are separately provided, the number of components is decreased and the dental care instrument 1 is more compact.

The head 30 includes the cooling air path 51 permitting cooling air to flow therein. Owing to such an arrangement, the cooling air which has flown in the cooling air path 51 and cooling water are mixed together in the meeting portion 52 and supplied to the surgical operation target site from the water supply opening 51 efficiently.

As described above, the head housing 31 included in the head 30 is formed by molding by use of metal stereolithography composite processing. Owing to such an arrangement, the head housing 31 having a complicated shape is formed with no provision of an unnecessary opening or the like.

This will be described in more detail. In the case where the cooling path 50 enclosing the tool holding portion 40 is formed by general drilling or the like, drilling needs to be performed linearly. Therefore, a plurality of linear through-holes are formed in combination. In this case, an unnecessary opening is formed. In the case where metal stereolithography composite processing is used, for example, even the head 30 including the cooling path 50 enclosing the tool holding portion 40 is formed by molding with no provision of such an unnecessary opening.

In the case where, for example, the cooling path 50 is formed by incorporating a component different from the components included in the head 30, which is desired to be compact, with the head 30, a tiny hollow member needs to be formed with high precision and to be incorporated with high precision such that cooling water flowing in the head 30 does not leak. In the case where the cooling path 50 is formed in the head housing 31 by molding by use of metal stereolithography composite processing, the number of components is decreased and the structure is suppressed from being complicated as compared with the case where such a different component is incorporated.

The cooling path 50 of the head housing 31 formed by, for example, metal stereolithography composite processing is allowed to have a desired shape. Therefore, as compared with the case where the cooling path 50 enclosing the tool holding path 40 is formed by a combination of a plurality of linear through-holes, the path has a lower path resistance. This increases the path efficiency of the cooling path, and thus increases the efficiency of cooling by the cooling water.

The head housing 31 is formed by stereolithography composite processing. Therefore, the head housing 31 including the cooling path 50 formed to have a cross-sectional diameter of 0.6 mm or less and the cooling air path 51 that is porous with microscopic pores is formed with high precision. Since the cooling air path 51 is porous with microscopic pores, the strength of the head housing 31 is prevented from being decreased due to the formation of the cooling air path 51.

In the above description, the cooling path 50 is formed in the thick wall of the head housing 31. Alternatively, the cooling path 50 may be formed by a component different from the components included in the head housing 31. Even in this case, the head housing 31 may be formed by metal stereolithography composite processing in the state of including the cooling path 50 formed of such a different component. In this manner, the cooling path 50 formed of such a different component is formed in the head housing 31 with high precision with no step of incorporating the cooling path 50 separate formed.

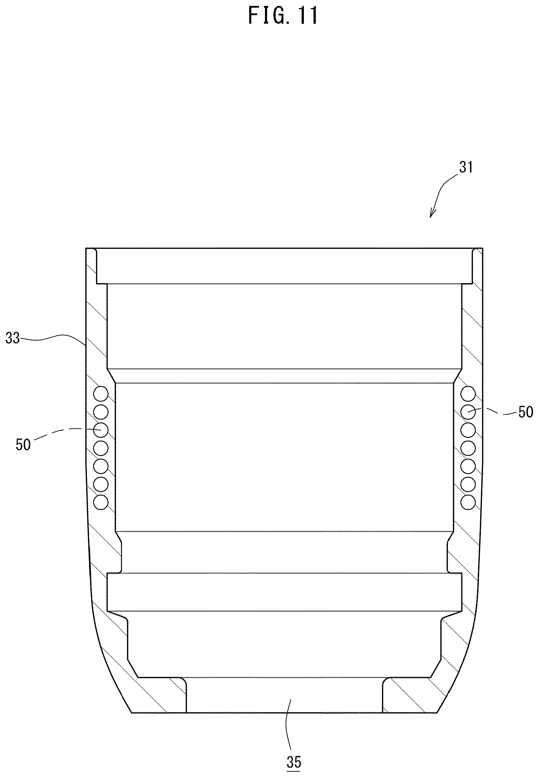

In the above description, the cooling path 50 having a long vertical cross-section is provided in the thick wall of the head housing 31. Alternatively, as shown in FIG. 11, a plurality of branch paths may be provided parallel to each other in the axial direction of the cutting rotation tool 5 to form the cooling path. The cooling path including such a plurality of branch paths has an increased surface area size. This increases the thermal exchange efficiency with the cooling water flowing in the cooling path, and thus increases the efficiency of cooling the head 30. The branch paths may be formed spirally around the axis of the cutting rotation tool in a circumferential portion of the head 30 so that water is supplied from the water supply opening at the end of the cooling path. Provision of the branch paths in such a spiral manner is encompassed in the provision of the branch paths parallel to each other. In FIG. 11, the cooling path is formed in a part, in an up-down direction, of a circumferential wall of the head 30. Alternatively, the cooling path may be formed in the entirety, in the up-down direction, of the circumferential wall. In FIG. 11, the cooling path is formed in the circumferential portion of the head 30. Alternatively, the cooling path may be formed in the neck 21. In the case of a straight handpiece, there is no portion corresponding to the neck 21. In this case, the cooling path may be formed in the grip as well as the head.

The cooling air path 51 may be porous with microscopic pores. In this case, the strength of the head 30 is prevented from being decreased due to the provision of the cooling air path 51.

The tool holding portion 40 may be of a cartridge type detachable from the head 30. In this case, the cooling path 50 may be provided between an outer surface of the tool holding portion 40 and an inner surface of a portion, of the head 30, to which the tool holding portion 40 is to be inserted.

The present invention is not limited to having the above-described structure, and may be carried out in many other embodiments. For example, the cooling water does not need to be supplied from the water supply opening 34. The cooling path 50 may be configured such that the cooling water is circulated and used only for cooling.

The handpiece is not limited to being of a contra-angle type, with which the cutting rotation tool 5 is attached in a direction crossing the longitudinal direction of the dental care instrument 1. Alternatively, the handpiece may be a straight handpiece, with which the cutting rotation tool 5 is attached coaxially with the longitudinal direction of the dental care instrument 1.

In the above description, the head housing 31 of the head 30 included in the dental care instrument 1 is formed by metal stereolithography composite processing described above. Another component included in the dental care instrument 1 may be formed by metal stereolithography composite processing. The dental care instrument 1, the motor unit 10, or the entirety of the contra-angle handpiece 20 may be formed by metal stereolithography composite processing.

In the above example, the stereolithography composite processing is performed by use of a metal material. Alternatively, stereolithography composite processing may be performed by use of a ceramic material or a resin material instead of the metal material.

In the above description, the stereolithography composite processing is performed by use of a metal material. Alternatively, stereolithography composite processing may be performed by use of a ceramic material or a resin material. In the case where a ceramic material or a resin material is for the stereolithography composite processing, the dental care instrument or the like is made lightweight.

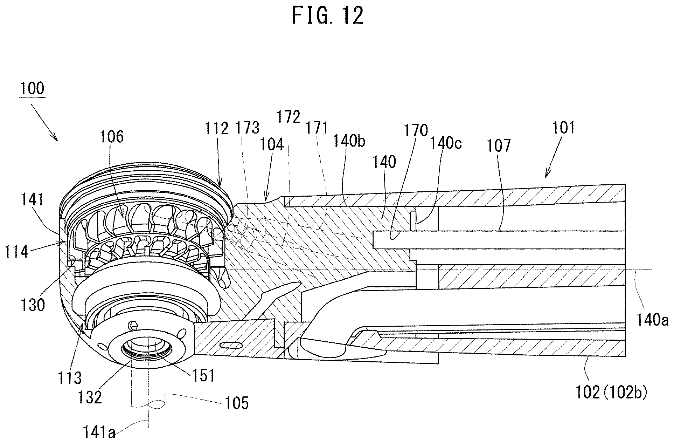

The above description is regarding the dental care instrument 1 including the cooling path 50 provided in the head 30. The present disclosure is not limited to this. Another dental care instrument or a medical care instrument may be formed by stereolithography composite processing. As an example thereof, a dental air turbine handpiece 101 shown in FIG. 12 through FIG. 14 will be described. The method for forming the dental air turbine handpiece 100 by molding by use of stereolithography composite processing, and functions and effects thereof, are the same, or substantially the same, as described above, and thus will not be described below.

The dental air turbine handpiece 101 includes a grip 102, which is to be gripped by an operator with his/her hand for performing a treatment. At a base end of the grip 102, a connection portion (not shown) is provided that is connected with a working medium supply tube 103 for a working medium such as air, water or the like, like a conventional handpiece. To a tip end of the grip 102, a head 104 is coupled via a neck 102b. To the head 104, a cutting tool 105 is detachably attached via a tool holding portion (not shown).

The head 104 includes a shaft 140 coupled with the tip end of the grip 102, and a cylindrical housing 141 accommodating the cutting tool 105 and a cutting tool driving portion 106, which drives the cutting tool 105. The shaft 140 and the cylindrical housing 141 are integrally formed. A rotation shaft 141a as a central shaft of the cylindrical housing 141 is directed vertical or substantially vertical with respect to a shaft core 140a of the shaft 140.

The shaft 140 of the head 104 includes a cross-section decreased portion 140b, which is processed to have such a size and shape as to be inserted and secured to the tip end of the cylindrical grip 102.

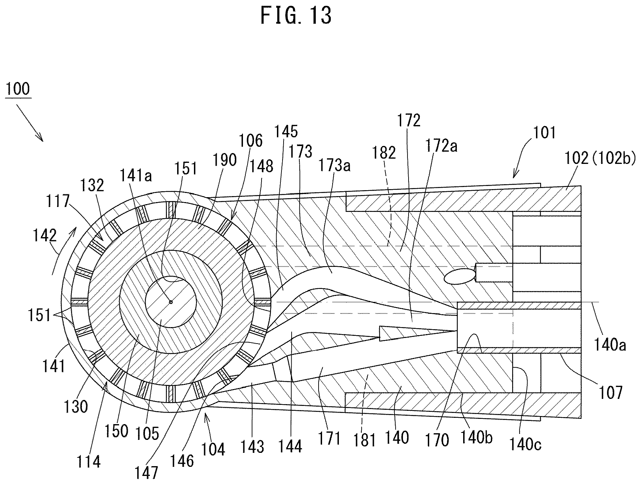

The shaft 140 has a plurality of through-holes formed therein. The plurality of through-holes fluidably communicates a rear end surface 140c on the grip 102 side (right end surface in FIG. 12 and FIG. 13) and an inner surface of the cylindrical housing 141.

The plurality of through-holes include air supply paths 171 through 173 usable to supply pressurized air to the cutting tool driving portion 106 and air discharge paths 181 and 182 usable to discharge the pressurized air from the cutting tool driving portion 106.

The air supply paths 171 through 173 are connected with an air supply pipe 107. The air supply pipe 107 is extended inside the grip 102 along a longitudinal direction of the grip 102 from a connection portion with the working medium supply tube 103.

As shown in FIG. 13, the air supply paths 171 through 173 are formed by molding at the same time as the shaft 140. Therefore, as compared with the case where being formed by forming holes having a predetermined length from the rear end surface 140c on the grip 102 side and an outer circumferential surface of the cross-section decreased portion 140b toward the inner surface of the cylindrical housing 141, the air supply paths 171 through 173 are smooth and have a low path resistance.

The air supply path 171 is in communication with a connection portion 170 of the air supply pipe 107 formed in the shaft 140. The air supply paths 172 and 173 are in communication with the connection portion 170 via communication paths 172a and 173a formed in the shaft 140.

Therefore, the pressurized air supplied from the air supply pipe 107 is introduced into the cylindrical housing 141 via the air supply paths 171 through 173. In this example, the communication path 173a is formed to be in communication with the air supply path 172 and also with the air supply path 173.

The air supply paths 171 through 173 are respectively provided with small diameter nozzles 143, 144 and 145 at tips thereof. Tips of the nozzles 143, 144 and 145 are opened toward the inner surface of the cylindrical housing 141 as air supply openings 146, 147 and 148.

Preferably, the nozzles 143, 144 and 145 are located such that the cutting tool 105 located inside the cylindrical housing 141 receives, by the pressurized air jetting out from the nozzles 143, 144 and 145, a rotation force acting in a direction of arrow 142 (clockwise direction in FIG. 13) centered around the rotation shaft 141a.

The nozzles 143, 144 and 145 are respectively linked with the air supply paths 171 through 173 via tapered portions. The nozzles 143, 144 and 145 are formed such that a total of cross-sectional area sizes of the linking portions via the tapered portions of the air supply paths 171 through 173 is larger than a total of cross-sectional area sizes of the nozzles 143, 144 and 145 opened at the inner surface of the cylindrical housing 141.

As shown in FIG. 12, the cylindrical housing 141 of the head 104 includes a cylindrical hollow portion 130. The cylindrical hollow portion 130 has a shape and a size corresponding to an outer shape of the cutting tool driving portion 106, which converts the pressurized air jetting out from the air supply paths 171 through 173 into the rotation force rotating the cutting tool 105. The hollow portion 130 is opened toward a top opening (not shown) thereabove and a bottom opening 132 therebelow.

A tool support portion 150 provided in the cutting tool driving portion 106 is configured as follows: the cutting tool driving portion 106 is inserted into the hollow portion 130 through the top opening, and the cutting tool 105 is detachably attached, via the bottom opening 132, to the tool support portion 150 (described below) provided in the cutting tool driving portion 106 inserted into the hollow portion 130.

The cutting tool driving portion 106 includes the tool support portion 150 supporting the cutting tool 105 along rotation shaft 141a in the hollow portion 130. The tool support portion 150 has a hole (tool support hole 151) having a predetermined depth from an end of the tool support portion 150. The tool support portion 150 includes a chuck mechanism (not shown) holding the cutting tool 105 inserted into the tool support hole 151.

The tool support portion 150 is supported by a top bearing 112 and a bottom bearing 113 respectively provided in a top portion and a bottom portion so as to be rotatable as centered around the rotation shaft 141a.

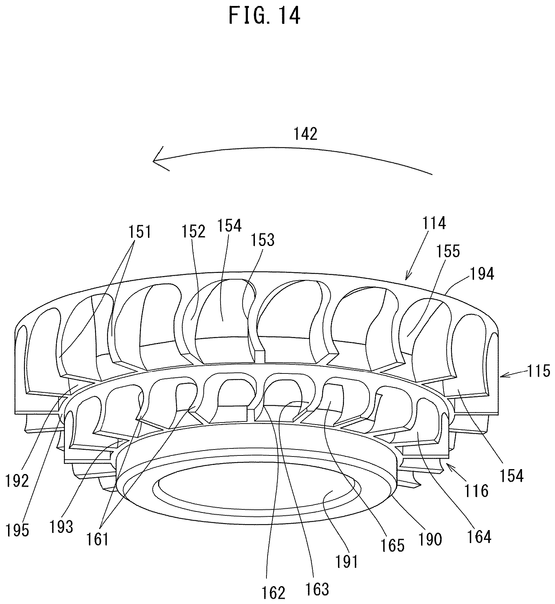

Now, a structure of a rotor 114 located inside the tool support portion 150 will be described with reference to FIG. 14.

In a portion of the tool support portion 150 between the top bearing 112 and the bottom bearing 113, the two-stage rotor 114 is integrally provided. The two-stage rotor 114 rotates the tool support portion 150 and the cutting tool 105 by use of the pressurized air jetting out from the air supply paths 171 through 173. In this case, the cutting tool 105 is rotated around the rotation shaft 141a via the tool support portion 150.

As shown in FIG. 14, the rotor 114 includes a ring-shaped hub 190. The ring-shaped hub 190 includes a central through-hole 191, an upper-stage large-diameter ring portion 192, and a lower-stage small-diameter ring portion 193. The upper-stage large-diameter ring portion 192 and the lower-stage small-diameter ring portion 193 are concentric with each other. An inner diameter of the through-hole 191 is substantially equal to an outer diameter of a substantially middle portion of the tool support portion 150 supporting the rotor 114. The middle portion of the tool support portion 150 is inserted into the through-hole 191 in a pressurized state to integrate the rotor 114 and the tool support portion 150. Alternatively, the rotor 114 and the tool support portion 150 may be integrally formed by metal stereolithography composite processing.

The large-diameter ring portion 192 and the small-diameter ring portion 193 provided on two, namely, the upper and lower stages are integral with each other. A first turbine blade 115 is provided on an outer circumferential surface of the upper-stage large-diameter ring portion 192, and a second turbine blade 116 is provided on an outer circumferential surface of the lower-stage small-diameter ring portion 193.

The first turbine blade 115 includes a circular top ceiling wall 194 formed at a top end of the hug 190, and a great number of (in this example, 18) protrusion walls (first turbine wings 151) extending downward from a bottom portion (bottom surface) of the top ceiling wall 194 along the outer circumferential surface of the large-diameter ring portion 192 and protruding radially externally.

The first turbine wings 151 are located at an equal interval along the outer circumferential surface of the large-diameter ring portion 192. Each of the first turbine wings 151 includes one surface (working surface 152 located upstream in the rotation direction 142 of the rotor 114) and the other surface (guide surface 153 located downstream in the rotation direction 142 of the rotor 114). Between two adjacent first turbine wings 151, a first air passage 154 is formed by the working surface 152, the guide surface 153, the bottom surface of the top ceiling wall 194 and the outer circumferential surface of the large-diameter ring portion 192.

The position in the height direction of the first air passage 154 (position in the height direction along the rotation shaft 141a) is set such that in the state where the rotor 114 is provided in the hollow portion 130, the pressurized air jetting out from the nozzles 143, 144 and 145 is blown into a top portion of the first air passage 154.

A portion of each first air passage 154 from the bottom surface of the top ceiling wall 194 to the outer circumferential surface of the large-diameter ring portion 192 is a curved surface 155 curved radially inward. The curved surface 155 is formed such that the air blown from the radially outer side of the rotor 114 into the first air passage 154 is smoothly changed in the direction downward along the curved surface 155 with the minimum possible air resistance. The curved surface 155 has a side shape that is concaved from the upstream side to the downstream side in the rotation direction 142 of the rotor as seen in a direction toward the rotation shaft 141a of the corresponding first turbine wing 151.

The second turbine blade 116 includes a bottom ceiling wall 195 having an outer edge defined by a bottom edge and an inner edge of the first air passage 154, and a great number of (in this example, 18) protrusion walls (second turbine wings 161) extending downward from a bottom portion of the bottom ceiling wall 195 along the outer circumferential surface of the small-diameter ring portion 193 and protruding radially outward.

The second turbine wings 161 are located at an equal interval along the circumferential direction. Each of the second turbine wings 161 includes one surface (working surface 162 located upstream in the rotation direction 142 of the rotor 114) and the other surface (guide surface 163 located downstream in the rotation direction 142 of the rotor 114). Between two adjacent second turbine wings 161, a third air passage 164 is formed by the working surface 162, the guide surface 163, the bottom surface of the bottom ceiling wall 195 and the outer circumferential surface of the small-diameter ring portion 193.

Especially, a portion of the third air passage 164 from the bottom surface of the bottom ceiling wall 195 to the outer circumferential surface of the small-diameter ring portion 193 is a curved surface 165 curved radially inward. The curved surface 165 is formed such that the air blown from the radially outer side of the rotor 114 into the third air passage 164 is smoothly changed in the direction downward along the curved surface 165 with the minimum possible air resistance.

The position in the height direction of the third air passage 164 (position in the height direction along the rotation shaft 141a) is set such that in the state where the rotor 114 is provided in the hollow portion 130, the bottom surface of the third air passage 164 is at substantially the same level as the air discharge paths 181 and 182. The curved surface 165 has a side shape that is concaved in the opposite direction to that of the first turbine wing 151 as seen in a direction toward the rotation shaft 141a of the corresponding second turbine wing 161. A bottom piece of each second turbine wing 161 is set to be directed toward the rotation direction 142.

An air introduction portion 117 usable to introduce the air from the first turbine blade 115 toward the second turbine blade 116 is provided in the hollow portion 130 of the head 104, in more detail, in a stage portion 138.

The pressurized air jetting out from the air supply paths 171 through 173 is guided to the inside of the first air passage 154 from the radially outer side thereof. The pressurized air discharged from a bottom end of the first air passage 154 is guided to the inside of the third air passage 164 from the radially outer side thereof.

The introduction portion 117 includes a plurality of (in this example, seven) second air passages 170 usable to guide the air from the bottom end of the first air passages 154 to the third air passages 164. The plurality of second air passages 170 are provided in a circumferential direction as centered around the rotation shaft 141a. A region 171, in which the plurality of second air passages 170 are provided, is on the entire circumference except for the side close to air discharge openings 156 and 157.

The region 171, in which the plurality of second air passages 170 are provided, does not include a region 74, in which the air supply openings 146, 147 and 148 of the air supply paths 171 through 173 are formed, and is larger than the region 74, in which the air supply openings 146, 147 and 148 are formed (FIG. 13) .

The plurality of second air passages 170 are formed from the stage portion 138 in the hollow portion 130 to an inner circumferential surface of a small-diameter cylindrical portion 137. The stage portion 138 and small-diameter cylindrical portion 137 are formed such that in the state where the cutting tool driving portion 106 is accommodated in the hollow portion 130, the first turbine blade 115 of the rotor 114 is fit in a large-diameter cylindrical portion 136 with a small gap, and the second turbine blade 116 is fit in a small-diameter cylindrical portion 137 with a small gap. The air supply openings 146, 147 and 148 are located so as to be opened toward an inner surface of the large-diameter cylindrical portion 136 and face the first air passages 154. The air discharge openings 156 and 157 are opened toward an inner surface of the small-diameter cylindrical portion 137 and are located at bottom ends of the third air passages 164.

At the positions of the stage portion 138 corresponding to the air discharge paths 181 and 182, a lengthy assisting discharge opening 83 in communication with the air discharge paths 181 and 182 is opened. Owing to this, a part of the air introduced into the first air passages 154 is not directed toward the second or third air passages 170 or 164 and is directly discharged from the air discharge paths 181 and 182.

As seen from the above, the unique structures of the first and second turbine wings 151 and 161 included in the rotor 114 and the unique structure of the second air passages 170, because of the combined effect thereof, decrease the rotation rate of the rotor 114 while maintaining the torque. In the dental air turbine handpiece 100, the rotor 114 and the head 104 are formed by metal stereolithography composite processing, and thus are formed to have a desired shape that increases the flowability of the pressurized air.

The air supply paths and the like are formed by stereolithography composite processing. Therefore, for example, the air supply paths that are highly airtight are formed with no need to insert, in a pressurized state, for example, a spherical sealing member such as a steel sphere or the like to close the openings of the air supply paths and the vicinity thereof.

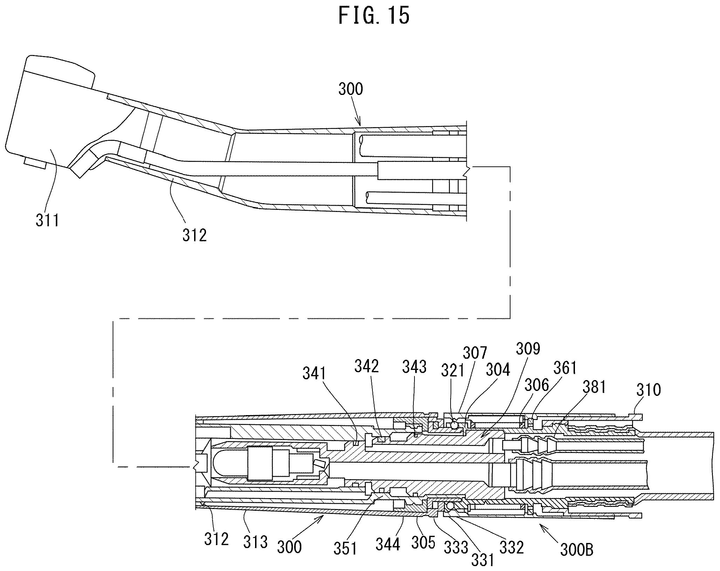

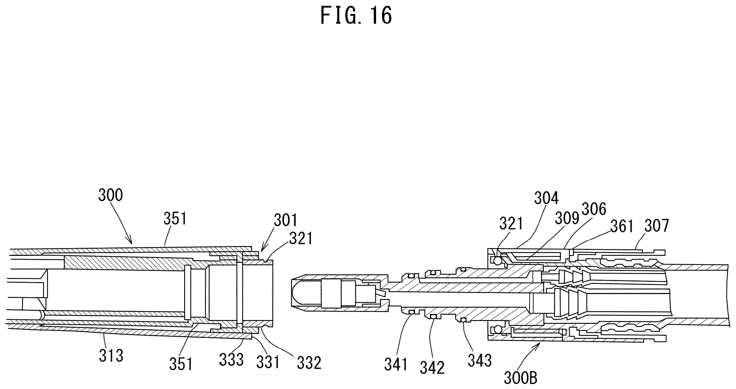

Now, with reference to FIG. 15 and FIG. 16, a dental air turbine handpiece will be described, in which a ball bearing is located at a connection end of a grip via a ball accommodated at a connection end of a joint in a circumferential direction.

A grip 300 includes a body 312 provided with a head 311 at a tip thereof, and a body cover 313. In the body cover 313, working supply members such as an air supply pipe, a water supply pipe, an illumination light assembly, a glass fiber and the like are accommodated in a grip-side joint 351. The body cover 313 encloses the working supply members. A rear end of the body 312 and a tip end of the grip-side joint 351 are fit into each other. The rear end of the body 312 is in engagement with the body cover 313 via an O-ring.

In the state where the grip 300 and a joint member 300B are connected with each other, a joint member-side joint 309 is accommodated in the grip-side joint 351. The joint member-side joint 309 allows air and water to be supplied and also allows the air to be discharged while being kept airtight by O-rings 341, 342, 343 and 344. The joint member-side joint 309 is relatively rotatable. A rear end of the joint member-side joint 309 is connected with air-supply, water-supply and air-discharge pipes 381.

The joint member 300B accommodates a tube joint 310 connected with a flexible tube 308 engaged with a rear end of the joint member-side joint 309.

To the front of a rear-end large-diameter portion of the joint member-side joint 309, a ball holder 304 of a ball 321 is engaged. The ball 321 is connected with the joint member 300B and stops the joint member 300B from moving toward the grip 300 in a thrust direction. The ball 321, together with the joint member-side joint 309, is rotatably connected along a rotation joint annular groove 382 in a rotation joint 332 on the grip 300 side.

The ball 321 is pressed and put into engagement with the rotation joint annular groove 382 by an annular convexed portion of a cylindrical ball press 307, which is positioned by a compression spring 306 provided between a spring holding ring 361, loosely fit to an outer circumferential surface of the tube joint 310, and the ball holder 304.

A cross-sectional shape of the annular convexed portion of the ball press 307 has a flat apex. In the state where the grip 300 and the joint member 300B are connected with each other, the ball press 307 is positioned by the compression spring 306 such that the ball 321 contacts the flat apex of the convexed portion. When the ball press 307 fit into the spring holding ring 361 against an elastic force of the compression spring 306 is moved forward or backward in the thrust direction of the handpiece, the convexed portion of the ball press 307 is moved forward or backward with respect to the ball 321. A gap in which the ball 321 is movable radially outward is formed between the ball 321 and the ball press 307. Regardless of whether the ball press 307 is moved forward or backward, the ball 321 is moved radially outward from the rotation joint annular groove 382 of the rotation joint 332. Therefore, the joint member 300B is detachable.

A connection end 301 of the grip 300 engages a secured collar 305 on the inner side of an rear end of the body cover 313, fits the ball 331 to the rotation joint 332 having an L-shaped cross-section, and wraps the ball 321 together with the rotation joint 332. The ball 331 and a ball guide 333 having an L-shaped cross-section are combined to become a combined ball bearing 303. An external groove 383 in an end surface of the ball guide 333 is used to screw the ball bearing 303 into the body cover 331 until contacting the secured collar 305 to assemble the joint member 300B to the connection end 301.

The rotation joint 332 of the connection end 301 thus assembled linearly bears an external force in a radial direction and a thrust direction of the joint by the ball 331 without being bonded with any member. Namely, the external force in the radial direction is linearly borne by the ball 331 by the external groove 383 of the rotation joint 332 and an inner circumferential surface of the ball guide 333. The external force in the thrust direction is linearly borne between flange end surfaces of the rotation joint 332 and the ball guide 333.

The grip 300 having such a structure, the body cover 313 included in the grip 300, and tiny operating components connecting the joint portion 300 and the joint member 300B such as the ball holder 304, the ball press 307 and the like, are formed by stereolithography composite processing. Owing to such an arrangement, these components are formed to have a desired shape with high precision. Therefore, for example, the operability is improved; for example, the handpiece is made easier to handle, or the rotation in the radial direction is made significantly smoother.

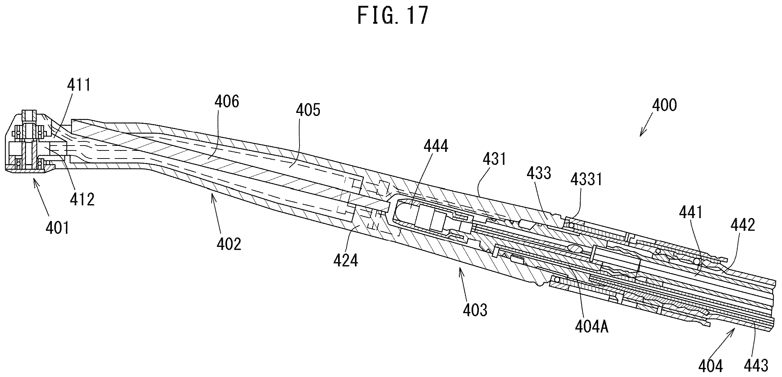

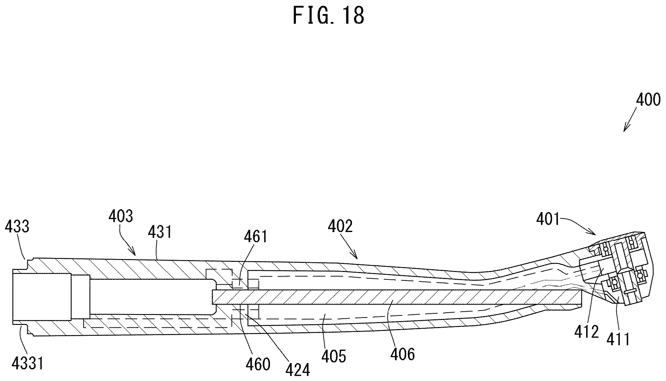

Now, a dental air turbine handpiece 400 shown in FIG. 17 and FIG. 18 will be described.

The dental air turbine handpiece 400 includes a head 401 accommodating a rotatable member 411 including a turbine wing 412, a grip 402 linked with the head 401, a joint 403 linked with the grip 402, and a working medium conduit 404 provided in an inner space of the joint 403 and detachably engaged and secured to the joint 403. The working medium conduit 404 is usable to supply various working mediums to the dental handpiece.

The grip 402 and the joint 403 linked with the grip 402 are integrally formed by stereolithography composite processing. A sealing portion 424 is formed between the grip 402 and the joint 403, and thus an inner space of the grip 402 and an inner space of the joint 403 are demarcated by the sealing portion 424.

A basic inner structure of the dental handpiece 400 is substantially the same as that of a conventionally used common handpiece, and includes a working medium passage for pressurized air for driving and rotating the turbine wing 412, pressurized water for cooling a site to be treated, or the like, and a light guide illuminating a damaged site.

This will be described more specifically. The dental handpiece 400 shown in FIG. 17 includes a working medium passage 404 (pipe, duct) including an air supply pipe 441, an air discharge pipe 442, and a water supply pipe 443, and also includes a straight light guide 406 located in the grip 402 for illuminating a damaged site.

In the dental handpiece 400, the working medium conduit 404 is provided inside the joint 403 from a read end of the joint 403 and is detachably engaged. The working medium conduit 404 is detachably engaged and secured to the joint 403. When being engaged with the joint 403, the working medium conduit 404 cooperates with an inner mechanism of the joint 403 to exert a function of allowing each of various working mediums to flow in a working medium conduit provided inside the grip 402 in correspondence with the respective working medium, and supplying or discharging the working medium to a proper site.

The light guide 406 provided inside the grip 402 is illuminated with illumination light emitted from an illumination light bulb 444 in the working function conduit 404.

The light guide 406 is formed of a straight (linear) glass rod, which is light-guiding. Other examples of the light-guiding materials include a glass fiber bundle, a plastic fiber bundle, a glass rod coated with a reflective film, and the like.

As shown in FIG. 17, the light guide 406 formed of a straight glass rod is provided so as to have a space therearound. In this case, the refractive index of the space (air) is smaller than the refractive index of the light guide 406 formed of a glass rod. Therefore, the light guide 406 formed of a glass rod is allowed to have the same structure as that of a total reflection-type clad glass fiber, and thus provides a splendid light transmission system having a very small attenuation ratio of light transmission.

As described above, among the head 401, the grip 402, and the joint 403 included in the dental handpiece 400, the grip 402 and the joint 403 are integrally formed by molding by use of stereolithography composite processing. The dental handpiece 400 including the head 401 is formed by separate members formed of a plurality of integrally moldable materials. Such separate members are joined together to be integral.

As a component of the dental handpiece 400, a blocking plate 405 is provided as a demarcating plate demarcating the inside of the dental handpiece 400 into a left part and a right part.

The blocking plate 405 has a concaved portion for holding the light guide 406 in order to stably hold and secure the light guide 406.

As shown in FIG. 18, the sealing portion 424 is formed between the grip 402 and the joint 403. As shown in the figure, the sealing portion 424 has communication holes 461 communicated with the working medium passages and a light guide holding hole 460 holding the light guide 406 at an axial position of the grip 402.

The rotatable member 411 formed by stereolithography composite processing with high precision is integrally incorporated into the head. In the head 401, the rotatable member 411 includes the turbine wing 412 as a main component. The turbine wing 412 is provided in a cylindrical chamber of the head 401 and is integrally provided with a rotatable shaft 413. To a tip end of the rotatable shaft 413, various tools such as a cutting bar and the like are detachably attached.

As shown in the figure, a tip end 451 of the blocking plate 405 includes an air supply opening 452, a total of two air discharge openings (453, 454) provided close to, and above and below, the air supply opening 452, and a water supply guide portion 455. The air supply opening 452 is in communication with an air supply passage 421, and the water supply guide portion 455 is in communication with a water supply passage 423. The two air discharge openings (453, 454) are in communication with an air discharge passage 422. This is why the two air discharge openings (453, 454) are located close to, and above and below, the air supply opening 452.

The dental handpiece 400 includes the light guide 406 guiding illumination light to an exit opening in a linear (straight) manner. The blocking plate 405 includes a light guide holding groove 456 guiding the illumination light in a linear manner.

A holding mechanism for the light guide 406 is provided by the light guide holding groove 456 of the blocking plate 405. The light guide 406 is located in the light guide holding groove 456. Both of two ends of the light guide holding groove 456 are bonded, whereas an outer circumferential surface thereof other than the bonded portions is exposed to air (discharged air). Therefore, as described above, the light guide 406, which is formed of one straight glass rod, prevents the illumination light from being attenuated. A reason for this is that the refractive index of glass is larger than the refractive index of air, and therefore, the light transmission system is like a clad glass fiber system.

The joint 403 is integrally formed with the grip 402 by molding by use of stereolithography composite processing. The joint 403 includes the sealing portion 424, a main body 431, and an engageable portion 433. The sealing portion 424 has the communication holes 461 communicated and connected with the working medium passages provided inside the grip 402. The main body 431 has communication holes 432 accommodating a tip end 404A of the working medium conduit 404 and cooperates with the working medium conduit 404 to form a working medium conduit path. The engageable portion 433 is disengageably engaged with an engageable portion of the working medium conduit 404 (not shown).

The sealing portion 424 includes the three communication holes 461 (461a through 461c) communicated with the air supply passage 421, the air discharge passage 422 and the water supply passage 423, which are working medium passages inside the grip 402.

The communication holes 461 (461a through 461c) are in communication with an air supply passage 481, an air discharge passage 482, and a water supply passage 483. The sealing portion 424 has a holding hole 434, holding the light guide 406, at a central portion thereof. The communication holes 461 are located in the sealing portion 424 at a desired interval in a circumferential direction.

The communication holes 432 formed inside the main body 431 of the joint 403 are opened at an end thereof and are configured to accommodate a tip end of the working medium conduit 404, namely, a working medium conduit-side joint (404A) (see FIG. 17).

The three communication holes 432 having a larger diameter stepwise from the sealing portion 424 toward the engageable portion 433 are respectively in communication with the communication holes 461 of the sealing portion 424 and are also respectively in communication with the working medium passages (441, 442, 443) of the working medium conduit 404.

In the example shown in the figures, the engageable portion 433 engageable with the working medium conduit 404 of the joint 403 includes an engagement annular groove 4331. The engagement annular groove 4331 is a connection portion detachable from the working medium conduit 404. In the example shown in the figures, the engagement annular groove 4331 is provided in an outer circumferential portion of the engageable portion 433. An engageable portion (spring steel + engageable steel sphere) of the working medium conduit-side joint 404A (see FIG. 17) is disengageably engaged with the engagement annular groove 4331, and thus the joint 403 and the working medium conduit 404 are relatively rotatable.

The joint 403 is integrally formed by molding by use of stereolithography composite processing, and includes, therein, communication holes 432, namely, an air supply passage an air discharge passage, and a water supply passage communicable with the working medium passages (441, 442, 443) of the working medium conduit 404.

As described above, the rotatable member 411 integrally incorporated into the head, the grip 402 including the blocking plate 405, the joint 403 including, for example, the sealing portion 424 having the communication holes 461, or the like is formed by stereolithography composite processing. Owing to such an arrangement, the highly precise components sufficiently exerting the respective functions are provided.

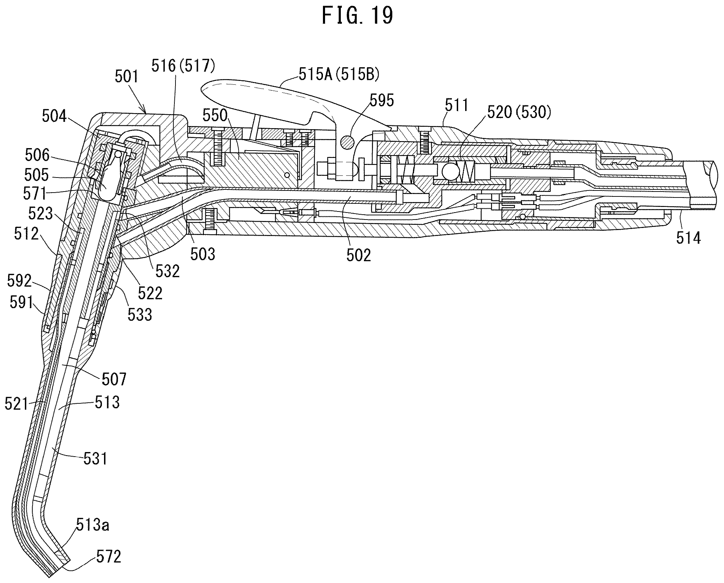

Now, with reference to FIG. 19, a three-way syringe will be described.

A handpiece main body 501 included in the three-way syringe includes a grip 511 and a nozzle 513 connected with the grip 511, via a tip end connection portion 512, so as to be bent with respect to the grip 511.

At a base end of the grip 511, a flexible tube 514 accommodating supply tubes for working mediums such as water, air and the like is coupled. The supply tubes are connected with supply paths 502 and 503 built in the grip 511. Therefore, the working mediums are sprayed toward a damaged site independently or in a mixed state from a tip end of the nozzle 513 via the supply paths 502 and 503 and supply paths 521 and 531 built in the nozzle 513.

The tip end connection portion 512 of the grip 511 is a female cylinder-like. At a bottom portion of the tip end connection portion 512, a cylindrical lamp holder 504 is provided coaxially with the nozzle 513. A lamp 505, which is an illumination light source, is detachably attached to the cylindrical lamp holder 504 via a lamp cartridge 506. The lamp 505 is attached in a direction of an optical axis thereof.

A light guide body (optical fiber) 507 is provided in the nozzle 513 along an axis of, and up to the tip end of, the nozzle 513. The nozzle 513 is configured such that when the nozzle 513 is connected with the grip 511, a light incident end 571 faces a front surface of the lamp 505 such that the damaged site is irradiated with the light of the lamp from an exit end 572 at a tip of the nozzle 513.

The nozzle 513 is connected with the grip 511 by an operation sleeve 591 so as not to be pulled out from the grip 511 and so as to be rotatable around an axis of the nozzle 513. The nozzle 513 is configured to be detachable from the grip 511 by operating the operation sleeve 591 of the tip end connection portion 512 against an elastic force of a compression spring 592.

A tip end 513a of the nozzle 513 is curved. The tip end 513a is directed in any direction in accordance with the damaged site because the tip end 513a has such a shape and the nozzle 513 is rotatable.

In the tip end connection portion 512, the grip 511 and the nozzle 513 are fit into each other in a female-male relationship. The supply paths 502, 521, 503 and 531 for the working mediums are in communication with each other via circumferential grooves 522 and 532 and communication holes 523 and 533 formed in the tip end connection portion 512.

The supply path 531, in the nozzle 513, provided for air is structure to be coupled with the nozzle 513 itself from the middle and to allow the air to be sprayed from an area around the supply path 521 for water at the tip end of the nozzle 513.

At an outer circumference of the grip 511, two operation levers 515A and 515B are swingably attached via a pin 595. The operation levers 515A and 515B are located at such an interval as to be operable at the same time by fingers. The operation levers 515A and 515B are operated such that when either one the operation levers 515A and 515B are pressed, or both thereof are pressed at the same time, a ball valve 520 and/or 530 provided in the middle of the supply paths 502 and/or 503 for the working mediums is opened and thus water and/or air is supplied to the nozzle 513.

The operation levers 515A and 515B are also configured such that when either one thereof is operated, a limit switch 550 located at a position corresponding to a position just below thereof is turned on or off, in accordance with which the lamp 505 is controlled to be turned up or off.

Inside the grip 511, a supply path 516 and a discharge path 517 for cooling air usable to cool the lamp cartridge 506 and the lamp 505 are provided. The supply path 516 is configured such that the cooling air is supplied to the supply path 516, among the paths 516 and 517, via an electromagnetic valve 560 outer to the device, which is opened or closed by the limit switch 550 being turned on or off in accordance with an operation made on the operation levers 515A or 515B. The electromagnetic valve 560 is controlled to be opened or closed by the lamp 505 being turned up or off.

As described above, in the tip end connection portion 512, the grip 511 and the nozzle 513 are fit into each other in a female-male relationship. The supply paths 502, 521, 503 and 531 for the working mediums are in communication with each other via the circumferential grooves 522 and 532 and the communication holes 523 and 533 formed in the tip end connection portion 512. Even the handpiece main body 501 having such a structure is formed with high precision by stereolithography composite processing.

With stereolithography composite processing, the two operation levers 515A and 515B swingably attached to the grip 511, and the grip 511, are processed together in the state where the operation levers 515A and 515B are attached to the grip 511. Therefore, the operation levers 515A and 515B and the grip 511 are formed with no step of being separately processed and then assembled.

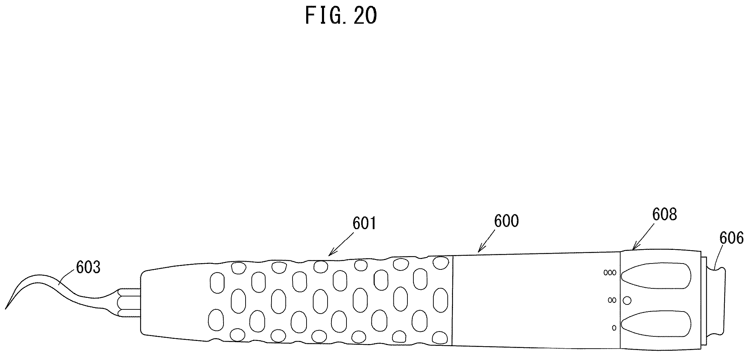

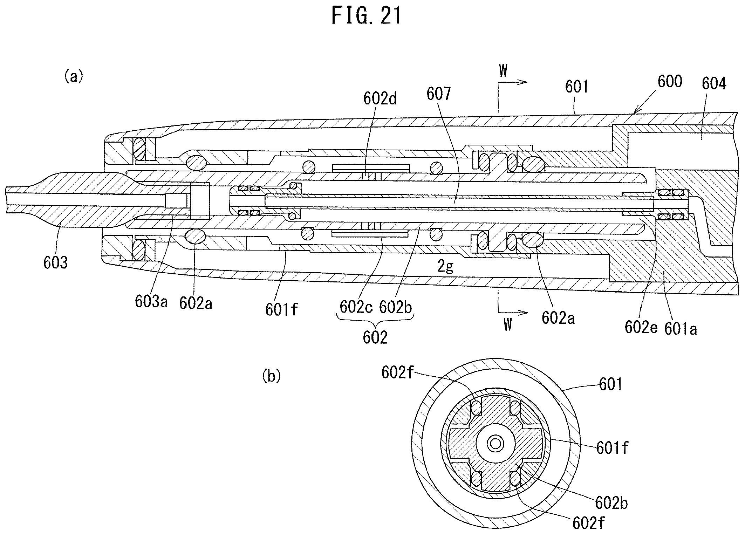

Now, a dental air scaler 600 shown in FIG. 20 and FIG. 21 will be described.

FIG. 20(a) is an external front view of the dental scaler 600 to which the present invention is applied. FIG. 21(a) is a vertical cross-sectional view of a main part including an air vibrator 602, and FIG. 21(b) is a cross-sectional view taken along line W-W in FIG. 21(a).

A handpiece main body 601 of the dental air scaler 600 is formed to have a substantially cylindrical grip shape so as to be easily gripped and operated with fingers. A core 601a of the handpiece main body 601 supports the air vibrator 602, acting as a driving portion, via a plurality of cushioning members 602a and 602f, which are elastic. The air vibrator 602 is supported so as to be capable of vibrating.

The air vibrator 602 includes a cylindrical vibrator main body 602b and a rotatable ring 602c loosely fit into an outer circumferential surface of the cylindrical vibrator main body 602b. The cylindrical vibrator main body 602b is opened at a base end 602e. The rotatable ring 602c is provided so as to cover an air ejection small hole 602d formed in a wall of the cylindrical vibrator main body 602b. At a tip end of the vibrator main body 602b, a scaler chip 603 is detachably attached.

In the core 601a of the handpiece main body 601, an air supply path 604 and an air discharge path 605 both for compressed air are formed. The air supply path 604 is in communication with the opened base end 602e of the vibrator main body 602b, and a start point of the air discharge path 605 is in an outer circumferential portion of the vibrator main body 602b.

The handpiece main body 601 is provided with a coupling portion 606 at a base end thereof. The coupling portion 606 is configured such that a driving medium supply hose accommodating a supply path and a discharge path that are linked with a compressor (not shown) are detachably attached to the coupling portion 606.

In the state where the hose is connected with the handpiece main body 601 via the coupling portion 606, the supply path and the discharge path in the hose are respectively connected with the air supply path 604 and the air discharge path 605 in the handpiece main body 601.

A tip of a supply water path 607 is extended inside the core 601a and the vibrator main body 602b and reaches an area in the vicinity of a tip end of the scaler chip 603. In the state where the handpiece main body 601 is connected with the hose via the coupling portion 606, the supply water path 607 is coupled with the water supply path accommodated in the hose. Therefore, water is supplied from the tip end of the scaler chip 603 toward a tooth or the like.

In the state where the handpiece main body 601 is connected with the hose via the coupling portion 606, the handpiece main body 601 is rotatable around an axis of each of a plurality of rolling balls 606a with respect to the hose. The plurality of rolling balls 606a are located in a circumferential direction, such that the operability of the handpiece main body 601, which is grasped and operated to perform a treatment or the like, is improved.

The compressed air supplied from the air supply path 604 is introduced from the opened base end 602e of the vibrator main body 602b into the cylindrical main body 602b, and the introduced compressed air jets out to the outside of the cylinder from the air ejection small hole 602d. At this point, the rotatable ring 602c is present on the ejection side in a loosely fit state, and therefore, is vibrated by the ejection action. At this point, the vibrator main body 602b is supported only by the cushioning members 602a and 602f, which are O-rings or the like, with respect to the core 601a of the handpiece main body 601. Therefore, the vibrator main body 602b is also vibrated at the same time by the reaction to the vibration of the rotatable ring 602c. This vibration is transmitted to the scaler chip 603 and thus is used to clean the surface of the tooth, remove the dental plaque or the like.

As described above, the compressed air subjected to the vibration of the air vibrator 602 is discharged via the air discharge path 605. In the middle of the air discharge path 605, an air discharge adjustment mechanism 608 is provided. The air discharge is adjusted and the output of the air vibrator 602 is adjusted by the air discharge adjustment mechanism 608.

The air discharge adjustment mechanism 608 is provided at the base end of the handpiece main body 601, so as not to be inadvertently touched by the operator while the operator grasps and operates the handpiece main body 601.

As described above, the air vibrator 602 includes the vibrator main body 602b, which has a cylindrical shape having a thin elliptical cross-section, and the rotatable ring 602c loosely fit into the outer circumferential surface of the vibrator main body 602b. The vibrator main body 602b is supported by the cushioning members 602a and 602f, which are O-rings or the like, with respect to the core 601a of the handpiece main body 601 and a cylindrical assisting member 601f linked with a tip of the core 601a. The air vibrator 602 is positioned to be concentric with the handpiece main body 601 and also positioned in a radial direction and a thrust direction by the cushioning members 602a and 602f. At a base portion of the scaler chip 603, a male screw 603a is formed. The scaler chip 603 is detachably engaged with the tip end of the vibrator main body 602b via the male screw 603a.

As described above, the handpiece main body 601 and the air vibrator 602 of the dental air scaler 600 are formed by stereolithography composite processing and thus with high precision. Therefore, the vibration of the vibrator main body 602b is transmitted to the scaler chip 603 efficiently, and thus the surgical operation is performed efficiently. In this example, the vibrator is the air scaler using the air vibrator. Alternatively, an ultrasonic scaler using an ultrasonic vibrator may be used.

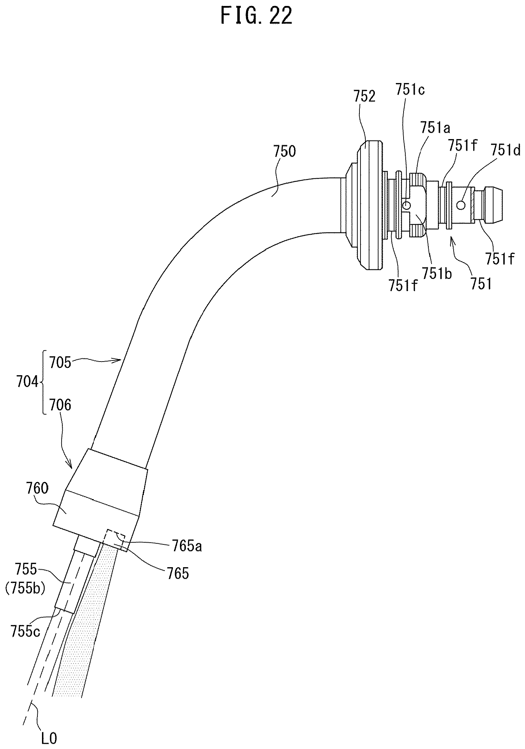

Now, a laser radiation handpiece including a laser radiation chip will be described with reference to FIG. 22 through FIG. 24.

A dental laser treatment device includes a laser device main body (not shown) including a laser oscillator oscillating laser light well absorbed by water, a water supply circuit supplying water, and an air supply circuit supplying air. The dental laser treatment device also includes a dental laser radiation handpiece 702, and flexible hose (not shown) connecting the laser device main body and the dental laser radiation handpiece 702 to each other, guiding laser light and supplying water, air and the like from the laser device main body to the dental laser radiation handpiece 702.

The flexible hose includes a bundle of a laser light guide body, a water supply tube and an air supply tube, and is configured to be connectable with a base portion of the dental laser radiation handpiece 702.

The laser oscillator located inside the laser device main body uses, as a laser generation source, medical-use laser well absorbed by water, such as Er:YAG laser, CO₂ laser, Er. Cr : YSGG laser, Ho:YAG laser or the like.

The dental laser radiation handpiece 702 includes a handpiece main body 720 including a grip connected with the flexible hose and gripped and operable by an operator with his/her fingers and a head linked with a tip end of the grip, and also includes a dental laser radiation chip 704 detachably attached to the head at a tip end of the handpiece main body 720.

In the state where the flexible hose and the handpiece main body 720 are connected with each other, the laser light guide body, the water supply tube and the air supply tube in the flexible hose are joined with base portions of a laser light guide path 721, a water path 722 and an air path 723 in the handpiece main body 720.

Owing to the above-described arrangement, the laser light guide body in the flexible hose is optically connected with the laser light guide path 721 in the handpiece main body 720, the water supply tube in the flexible hose is connected with the water path 722 in the handpiece main body 720 in a watertight state, and the air supply tube in the flexible hose is connected with the air path 723 in the handpiece main body 720 in an airtight state.

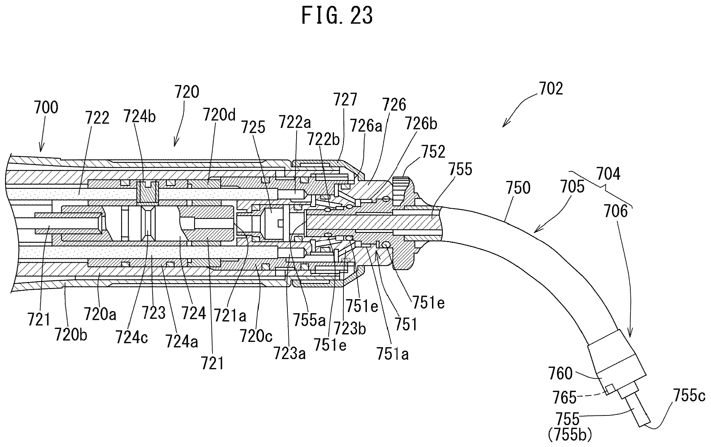

With reference to FIG. 22 and FIG. 23, the connection portion of the handpiece main body 720 and the dental laser radiation chip 704 will be described in detail.

As shown in FIG. 23, the handpiece main body 720 includes a main body cylindrical portion 720a extending from a grip 700B to the head, and an external sleeve 720b provided outside the main body cylindrical portion 720a. The laser light guide path 721 includes a hollow or solid wave guide path (fiber) and is provided concentrically with the handpiece main body 720 so as to extend in a longitudinal direction thereof. A tip end of the laser light guide path 721 is held by a ferrule 724 and faces the inside of the head.

The ferrule 724 is inserted into a support ring 724a, which is concentrically inserted into, and supported by, the main body cylindrical portion 720a. A tip of a securing screw 724b engaged with the support ring 724a is aligned with a circumferential groove 724c formed in a circumferential surface of the ferrule 724, and thus the ferrule 724 is positioned with respect to the support ring 724a.

The support ring 724a is inserted into, supported by, a predetermined position in main body cylindrical portion 720a via a spacer collar 720d by a cylindrical holder 720c engaged with a tip end of the main body cylindrical portion 720a. A tip end 721a of the laser light guide path 721 inserted into, and supported by, the ferrule 724 faces an optical collection lens 725 held by the holder 720c.

The water path 722 and the air path 723 are each formed of a thin tube. Tip ends of the water path 722 and the air path 723 are held by the holder 720c and are respectively in communication with a water communication path 722a and an air communication path 723a formed in the holder 720c. A chip holder 726 is fit into the holder 720c. The chip holder 726 puts a cap nut 727 into engagement with a tip end of the external sleeve 720b. Thus, the water path 722 and the air path 723,and the cap nut 727, are secured and maintained in a fit state.

The chip holder 726 is hollow and cylindrical. An inner cylindrical portion 726a formed concentrically with the chip holder 726 is a connection portion usable to detachably attach, by engagement, a chip main body 705 included in the dental laser radiation chip 704 to the chip holder 726. In a wall of the cylindrical chip holder 726, a water communication passage 722b and an air communication passage 723b are formed.

In the state where the chip holder 726 is fit and secured to the holder 720c, the water communication passage 722b and the air communication passage 723b are respectively linked with the water communication path 722a and the air communication path 723a via circumferential grooves formed in the outer circumferential portion of the chip holder 726.

Tip ends of the water communication passage 722b and the air communication passage 723b are opened toward the inner cylindrical portion 726a of the chip holder 726. In the inner cylindrical portion 726a of the chip holder 726, a female screw 726b usable to engageably join the chip main body 705 with the chip holder 726 is formed.

In FIG. 23, the water path 722, the air path 723, the water communication path 722a, the air communication path 723a, the water communication passage 722b, the air communication passage 723b, the securing screw 724b and the like are shown on the same cross-section for the sake of convenience. However, these are designed to be formed at appropriate positions in a circumferential direction so as not to interfere with each other.

In a connection portion of the chip main body 205 with the head of the handpiece main body 720, a bamboo sprout-shaped plug 751 inserted into, and engaged and connected with, the inner cylindrical portion 726a of the chip holder 726, and a knurling rotatable knob 752 engaged with the plug 751 to operate the plug 751, are integrally formed. In a circumferential surface of the plug 751, a male screw 751a engaged with the female screw 726b is formed. In a part of the male screw 751a, a milling-cut surface 751b is formed. The formation of the milling-cut surface 751b provides a gap between the male screw 751a and the female screw 726b, which are engageable with each other.

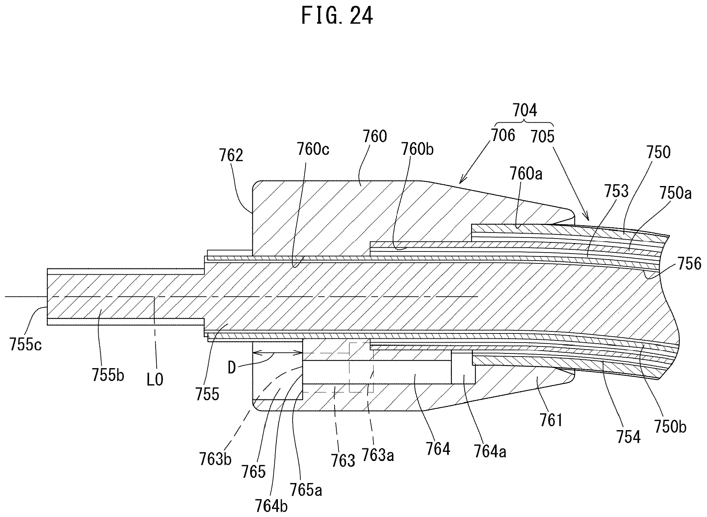

An air inlet 751c opened toward the gap is formed. The air inlet 751c is formed so as to be aligned with the air communication passage 723b in the state where the plug 751 is engaged and connected with the chip holder 726 and so as to extend in the plug 751 and communicate with an air flow path 754 (see FIG. 24) in an external pipe 750 described below.

At a position that is in a circumferential surface of the plug 751 and is separated from the air inlet 751c in an axial direction, a water inlet 751d is formed. The water inlet 751d is formed so as to be aligned with the water communication passage 722b when the plug 751 is engaged and connected with the chip holder 726 and so as to extend in the plug 751 and communicated with a water flow path 753 (see FIG. 24) described below in the external pipe 750.

To the circumferential surface of the plug 751, three O-rings 751e (see FIG. 23) are attached respectively via O-rings groove 751f (see FIG. 22). The O-rings 751e isolate the air communication portion and the water communication portion and are provided such that air communication and water communication through the air inlet 751c and the water inlet 751d are performed in an airtight state and a watertight state. A base end surface 755a of a fiber 755 faces a light output surface of the optical collection lens 725, and the laser light is allowed to pass the optical collection lens 725.

Now, with reference to FIG. 24, the dental laser radiation chip 704 will be described in more detail.

The dental laser radiation chip 704 includes the chip main body 705 formed of a curved rod-like body and a laser radiation tip member (chip tip end) 706 detachably attached to a tip end of the chip main body 705.

The chip main body 705 includes the external pipe 750, which is curved, an intermediate pipe 750a provided in, and substantially concentrically with, the external pipe 750, and an inner pipe 750b provided in, and substantially concentrically with, the intermediate pipe 750a.

A tip of the intermediate pipe 750a extends to protrude from a tip of the external pipe 750, and a tip of the inner pipe 750b extends to protrude from the tip of the intermediate pipe 750a.

The plug 751 and the knurling rotatable knob 752 are integrally formed with the external pipe 750. An annular gap between the external pipe 750 and the intermediate pipe 750a is the air flow path 754. An annular gap between the intermediate pipe 750a and the inner pipe 750b is the water flow path 753. An inner cylindrical portion in the inner pipe 750b is a fiber guide 756.

The fiber 755 inserted into the fiber guide 756, the water flow path 753 and the air flow path 754 are coaxial with each other, and are provided substantially parallel to each other and also to the chip main body 705.

The laser radiation tip member (chip tip end) 706 detachably attached to the tip end of the chip main body 705 is hollow and substantially cylindrical, and is formed of a molded boy 760 formed of a resin or the like. The laser radiation tip member (chip tip end) 706 includes an attach portion (linking portion) 761 detachably attached to, more specifically, outserted around, the external pipe 750 of the chip main body 705 in a pressurized state.

An inner cylindrical portion inside the molded body 760 includes a large-diameter cylindrical portion 760a having an inner diameter substantially equal to, or slightly smaller than, the outer diameter of the external pipe 750, a middle-diameter cylindrical portion 760b having an inner diameter substantially equal to the outer diameter of the intermediate pipe 750a, and a small-diameter cylindrical portion 760c having an inner diameter substantially equal to the outer diameter of the inner pipe 750b. The large-diameter cylindrical portion 760a, the middle-diameter cylindrical portion 760b, and the small-diameter cylindrical portion 760c are coaxial with each other and has an axis Lo of the molded body 760 as the axis thereof, and are formed to be continued in this order in a stepped state from the attach portion 761 toward a front end surface 762.

In the state where the laser radiation tip member 706 is attached to the chip main body 705, the large-diameter cylindrical portion 760a is outserted around the external pipe 750 in a pressurized state, the middle-diameter cylindrical portion 760b is outserted around the intermediate pipe 750a in an airtight state, and the small-diameter cylindrical portion 760c is outserted around the inner pipe 750b in a watertight state. A portion of the molded body 760 corresponding to the large-diameter cylindrical portion 760a substantially forms the attach portion 761.

In the state where the laser radiation tip member 706 is attached to the chip main body 705, the inner pipe 750b of the chip main body 705 is fit into the small-diameter cylindrical portion 760c, and extend to run through the laser radiation tip member 706, namely, the molded body 760 along the axis Lo and protrude from the front end surface 762 of the molded body 760.

Therefore, the fiber guide 756 provided by the inner cylindrical portion of the inner pipe 750b extends to the laser radiation tip member 706 and also forms a fiber guide for the laser radiation tip member 706.

The fiber 755 inserted into the fiber guide 756 protrudes from a tip end of the fiber guide 756. The protruding portion of the fiber 755 is held by the laser radiation tip member 706 concentrically therewith, in the state where an external casing called a "jacket" is removed and a core member 755b formed of a core and a clad or formed of only a core is exposed. A tip end surface of the core member 755d is a laser light output end 755c.

At a step portion between the small-diameter cylindrical portion 760c and the middle-diameter cylindrical portion 760b in the molded body 760, a water storage 763a in communication with the water flow path 753 is formed. A water communication path 763 extending from the water storage 763a toward the front end surface 762 parallel to the axis Lo is formed.

At a step portion between the middle-diameter cylindrical portion 760b and the large-diameter cylindrical portion 760a, an air storage 764a in communication with the air flow path 754 is formed. An air communication path 764 extending from the air storage 764a toward the front end surface 762 parallel to the axis Lo is formed in the vicinity of the water communication path 763. In the molded body 760, a concaved portion 765 opened toward the front end surface 762 and concaved toward the chip main body 705 side is formed. Tip end openings 763b and 764b of the water communication path 763 and the air communication path 764 face a bottom portion 765a of the concaved portion 765.

The laser radiation tip member 706 is formed by stereolithography composite processing and thus with high precision. Therefore, the optical collection lens 725 is located at an accurate position, and thus laser light is directed accurately and efficiently.

In the above example, the laser light source is connected with a portion outside the handpiece via the light guide path. Alternatively, the light source may be built in the handpiece.

In the present invention, the medial instrument component is a component forming a part of a medical instrument such as, for example, a medical handpiece, a medical laser handpiece or the like. Alternatively, the medial instrument component may be a medial handpiece having a built-in LED or semiconductor laser light source, a medical photopolymerization radiation device, or a medical light radiation device. In the LED, the semiconductor laser device, the medical photopolymerization radiation device, or the medical light radiation device, the light source acts as an operation portion.

The elements of the present invention and the elements of the above-described embodiment correspond to each other as follows.

The medical instrument element according to the present invention corresponds to the dental care instrument 1, the laser radiation device 100, the dental handpiece including the grip 300, the dental handpiece 400, the three-way syringe including the handpiece main body 501, the dental scaler 600, the dental laser radiation handpiece 702 including the dental laser radiation chip 704, the medical photopolymerization radiation device, the connection portion connecting the medical handpiece and the tube with each other, the head of the medical handpiece, the air turbine handpiece body of the medical handpiece, a bladed wheel, or a component included in the above. Similarly, the working medium corresponds to cooling water, water, air, air or laser light. The stack molding corresponds to the stereolithography composite processing. The present invention is not limited to having the structure of the above-described embodiment, but may be carried out in any of various embodiments. In the present invention, the stereolithography composite processing may be metal stereolithography composite processing using a metal powder as a stacking material. In this case, titanium or stainless steel powder, which is difficult to be processed by usual cutting, may be used.

For example, the stack molding may be, other than stereolithography composite processing, optical molding, powder sintering stacking, powder bonding, thermal melting stacking, sheet stacking, inkjet stacking or the like.

In the above-described description, for example, the head housing 31 in the contra-angle handpiece 20 is formed by stereolithography composite processing. Alternatively, the entirety of the contra-angle handpiece 20 may be formed by stereolithography composite processing. Still alternatively, components of the medical instrument or a part of, or the entirety of, the medical instrument may be formed by stereolithography composite processing. In this case, for example, the entirety of the contra-angle handpiece 20 is formed by stereolithography composite processing, but is formed in a divided manner in consideration that the contra-angle handpiece 20 is assembled with another member.

### REFERENCE SIGNS LIST

- 1 ...: Dental care instrument
- 100 ...: Laser radiation device
- 300 ...: Grip
- 400 ...: Dental handpiece
- 501 ...: Handpiece main body
- 600 ...: Dental air scaler
- 704 ...: Dental laser radiation chip
- 702 ...: Dental laser radiation handpiece

## Claims

1. A medical handpiece (400), comprising:
a medical instrument element comprising a medium passage (51) permitting a working medium to flow therein and including a bent portion at which a flow direction of the working medium is changed, the medium passage being porous with microscopic pores and the medical instrument element being formed by stack molding,
wherein the working medium is formed of a cooling medium;
the medium passage being a cooling path (51) that is located in the vicinity of an operation portion warmed by operating and cools the operation portion by the cooling medium flowing in the medium passage; and
the operation portion acting as a motive power transmission element (25, 26) transmitting a motive power or a guide element (406) guiding another member.

2. The medical handpiece according to claim 1, wherein the medical instrument element is formed by stereolithography composite processing used as the stack molding, the stereolithography composite processing being performed by a repetition of stereolithography and cutting processing.

3. The medical handpiece according to claim 1, wherein a difficult-to-cut metal material being used as a material for the medical instrument element, and wherein the difficult-to-cut material is a metal material considered to be difficult to be cut in usual cutting processing, such as for example titanium, stainless steel or the like.

4. The medical handpiece according to any one of claims 1 through 3, the medium passage being formed to have a cross-sectional diameter of 0.6 mm or less.

## Patentansprüche

1. Medizinisches Handgerät (400), aufweisend:
ein Element für ein medizinisches Instrument, das einen Medienkanal (51) aufweist, der gestattet, dass ein Arbeitsmedium darin strömt, und einen gebogenen Teil aufweist, bei dem eine Strömungsrichtung des Arbeitsmediums geändert wird, wobei der Medienkanal mit mikroskopischen Poren porös ist und das Element für ein medizinisches Instrument mittels Stack-Molding geschaffen ist,
wobei das Arbeitsmedium aus einem Kühlmedium besteht;
wobei der Medienkanal ein Kühlweg (51) ist, der in der Umgebung eines durch den Betrieb erwärmten Operationsteils gelegen ist und den Operationsteil durch das im Medienkanal strömende Kühlmedium kühlt; und
der Operationsteil als ein eine Antriebskraft übertragendes Element (25, 26) zur Übertragung einer Antriebskraft oder ein ein anderes Bauteil führendes Führungselement (406) dient.

2. Medizinisches Handgerät nach Anspruch 1, wobei das Element für ein medizinisches Instrument mittels einer als das Etagen-Formen bzw. Stack-Molding genutzten Stereolithografie-Verbundbearbeitung geschaffen ist, wobei die Stereolithografie-Verbundbearbeitung mittels einer Wiederholung einer Stereolithografie und einer Schneidbearbeitung durchgeführt wird.

3. Medizinisches Handgerät nach Anspruch 1, wobei ein schwer zu schneidendes Metallmaterial als Material für das Element für ein medizinisches Instrument verwendet wird und wobei das schwer zu schneidende Metallmaterial ein Metallmaterial ist, das als in einer herkömmlichen Schneidbearbeitung schwer zu schneiden gilt, wie etwa zum Beispiel Titan, rostfreier Stahl oder dergleichen.

4. Medizinisches Handgerät nach einem der Ansprüche 1 bis 3, wobei der Medienkanal so ausgebildet ist, dass er einen Querschnittsdurchmesser von 0,6 mm oder weniger hat.

## Revendications

1. Pièce à main médicale (400) comprenant :
un élément d'instrument médical comprenant un passage de fluide (51) permettant à un fluide de travail de s'écouler à l'intérieur et incluant une partie coudée où une direction d'écoulement du fluide de travail est modifiée, le passage de fluide étant poreux avec des pores microscopiques et l'élément d'instrument médical étant formé par moulage par empilement,
dans lequel le fluide de travail est formé d'un fluide de refroidissement ;
le passage de fluide étant une voie de refroidissement (51) qui est située à proximité d'une partie d'actionnement chauffée par actionnement et refroidit la partie d'actionnement par le fluide de refroidissement s'écoulant dans le passage de fluide ; et
la partie d'actionnement agissant comme un élément de transmission de puissance motrice (25, 26) transmettant une puissance motrice ou un élément guide (406) guidant un autre organe.

2. Pièce à main médicale selon la revendication 1, dans lequel l'élément d'instrument médical est formé par traitement composite de stéréolithographie utilisé comme moulage en grappe, le traitement composite de stéréolithographie étant exécuté par une répétition de traitement de stéréolithographie et de coupe.

3. Pièce à main médicale selon la revendication 1, dans lequel un matériau de métal difficile à couper est utilisé comme matériau pour l'élément d'instrument médical, et dans lequel le matériau difficile à couper est un matériau de métal considéré comme étant difficile à couper dans un traitement de coupe courant, comme par exemple le titane, l'acier inoxydable ou similaire.

4. Pièce à main médicale selon l'une quelconque des revendications 1 à 3, le passage de fluide étant formé pour avoir un diamètre de section transversale de 0,6 mm ou moins.
